# EUROPÄISCHE PATENTSCHRIFT

(11) **EP 1 637 533 B1**
(45) Veröffentlichungstag und Bekanntmachung des Hinweises auf die Patenterteilung: **13.08.2008**
(21) Anmeldenummer: 05026811.9
(22) Anmeldetag: 13.03.2002
(51) Int. Cl.: C07D 513/04, A61K 31/505, A61P 25/00, A61P 29/00

(54) **Substituierte Thiazolopyrimidine als Analgetika**
Substituted thiazolopyrimidines as analgesics
Thiazolopyrimidines comme médicaments analgésiques

(30) Priorität: 14.03.2001 DE 10112197; 29.10.2001 DE 10153344
(43) Veröffentlichungstag der Anmeldung: 22.03.2006
(62) Teilanmeldung aus: 02729998.1
(73) Patentinhaber: Grünenthal GmbH, 52099 Aachen (DE); Universiteit Leiden, 2300 RA Leiden (NL)
(72) Erfinder: Gerlach, Matthias, Dr., 63636 Brachttal (DE); Sundermann, Corinna, Dr., 52074 Aachen (DE); Jagusch, Utz-Peter, 52066 Aachen (DE); Sundermann, Bernd, Dr., 52074 Aachen (DE); Fuhr, Martin, 47918 Tönisvorst (DE); Ijzerman, Adriaan P., Prof., 2036MB Haarlem (NL); Dissen-De Groote, Miriam, 4336KB Middelburg (NL)

(56) Entgegenhaltungen:
- EP-A- 0 398 283
- US-A- 5 602 144

## Beschreibung

Die vorliegende Anmeldung betrifft substituierte Thiazolopyrimidine, Verfahren zu ihrer Herstellung, Arzneimittel, die diese Verbindungen enthalten, die Verwendung dieser Verbindungen zur Herstellung von Arzneimitteln zur Behandlung und/oder Prophylaxe von Schmerz, Epilepsie, Schizophrenie, neurodegenerativen Erkrankungen, insbesondere Morbus Alzheimer, Morbus Huntington und Morbus Parkinson, von cerebralen Ischämien und Infarkten, von Psychosen bedingt durch erhöhten Aminosäurespiegel, Hirnödemen, Unterversorgungszuständen des zentralen Nervensystems, insbesondere bei Hypoxien, insbesondere Neugeborenen-Hypoxie, und Anoxien, von AIDS-Demens, von Encephalomyelitis, des Tourette-Syndroms, der perinatalen Asphyxie, bei Tinnitus, neuropathischem Schmerz, Atemwegserkrankungen, Krebs, kardialen Arrythmien, Immunstörungen und -erkrankungen, Entzündungszuständen und -erkrankungen, neurodegenerativen Erkrankungen, Morbus Parkinson, Nierenversagen, Schizophrenie, Schlafstörungen, Schlaganfall, Thrombosen, Harninkontinenz, Diabetes, Psoriasis, septischem Schock, Gehirntraumata, Glaukom und/oder Stauungsinsuffizienz, sowie diese Verbindungen enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.
Opioide entfalten ihre analgetische Wirkung durch Bindung an zellmebranständige Rezeptoren, die zu der Familie der so genannten G-Proteingekoppelten Rezeptoren gehören. Neben diesen gibt es weitere Rezeptoren sowie Ionenkanäle, die wesentlich an dem System der Schmerzentstehung und der Schmerzweiterleitung beteiligt sind, beispielsweise der N-Methyl-D-Aspartat-Ionenkanal (NMDA-lonenkanal), über den ein wesentlicher Teil der Kommunikation von Synapsen abläuft und durch den der Calcium-lonenaustausch zwischen einer neuronalen Zelle und ihrer Umgebung gesteuert wird (s. z.B. P. D. Leeson, L. L. Iversen, J. Med. Chem. 37 (1994) 4053-4067).

Wichtige Erkenntnisse über die physiologische Bedeutung von ionenkanalselektiven Substanzen sind durch die Entwicklung der "patch-clamp"-Technik ermöglicht worden, mit deren Hilfe sich die Wirkung von NMDA-Antagonisten (d.h. Antagonisten des NMDA-Ionenkanals) auf den Calciumhaushalt im Zeilinneren nachweisen läßt.

Im nichtaktivierten Zustand sind die NMDA-lonenkanäle jeweils durch einzelne Magnesiumionen verschlossen, die sich im Inneren des Kanals befinden und diesen aufgrund ihrer Größe nicht passieren können. Im aktivierten Zustand können die kleineren Calcium- und Natriumionen den Kanal passieren. Die (+)-MK801-Bindungsstelle des NMDA-lonenkanals (ionotroper NMDA-Rezeptor) befindet sich ebenfalls im Inneren dieses Membranproteins. Substanzen mit NMDA-antagonistischer Wirkung, wie Phencyclidin (PCP), Ketamin oder MK801, besetzen diese Bindungsstelle (sogenannte "Channelblocker") und verschließen daher den betreffenden NMDA-lonenkanal.

Die EP 0 398 283 offenbart Substanzen mit einem kondensierten heterocyclischen Skelett und NMDA-antagonistischer Wirkung.

Der vorliegenden Erfindung liegt als eine Aufgabe zugrunde, analgetisch wirksame Verbindungen zur Verfügung zu stellen, die sich zur Schmerztherapie - ggf. auch zur Therapie chronischer und neuropathischer Schmerzen - eignen. Darüber hinaus sollten diese Substanzen möglichst keine der Nebenwirkungen, die üblicherweise bei der Anwendung von Opioiden wie Morphin auftreten, wie z.B. Übelkeit, Erbrechen, Abhängigkeit, Atemdepression oder Obstipation, hervorrufen.

Diese Aufgabe wird durch die Verbindungen der allgemeinen Struktur (II) gelöst, die analgetisch wirksam sind und an die MK801-Bindungsstelle des NMDA-Rezeptors binden. Bei den erfindungsgemäßen Verbindungen handelt es sich um substituierte Thiazolopyrimidine der allgemeinen Struktur (II) 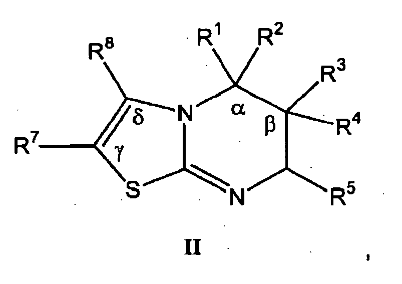 worin
- R¹ und R²: unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten, wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
- R³ und R⁴: H, Aryl' oder -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder
- einer der Reste R¹ und R²: zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 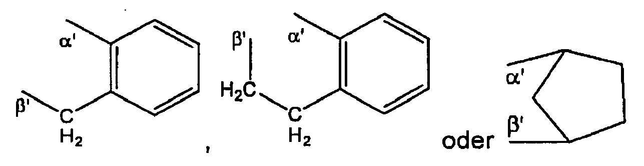 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
- R⁵: Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NO₂, -SH, -OH, -CO₂H und CO₂Alkyl; Aryl' oder -(CH₂)ₖ-Aryl', wobei k = 1,2,3 oder 4 ist, Heterocyclyl oder C(=O)R¹¹ bedeutet;
- R⁷: H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
- R⁸: H oder Aryl' bedeutet,
oder die Reste R⁷ und R⁸ zusammen Y bilden, wobei Y γ'-CR²¹=CR²²-CR²³=CR²⁴-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist;
- R⁹: unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
- R¹⁰: Aryl' bedeutet;
- R¹¹: Aryl' oder OR²⁵ bedeutet;
- R¹⁸: H oder Methyl bedeutet;
- R¹⁹: H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
- R²¹, R²², R²³ und R²⁴: unabhängig voneinander H, Fluor, Chlor, Brom, Iod oder OR²⁸ bedeuten;
- R²⁵: H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
- R²⁸: H, Methyl oder Ethyl bedeutet;
- Heterocyclyl: Furan-2-yl, Furan-3-yl, Thien-2-yl, Thien-3-yl, Pyridin-2-y-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂, -CO₂H und -CO₂Alkyl, bedeutet;
- Aryl': Aryl¹, Aryl² oder Aryl³ bedeutet;

- Aryl¹: für 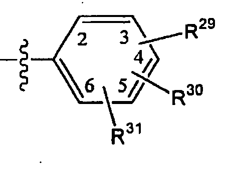 steht;
- Aryl²: für 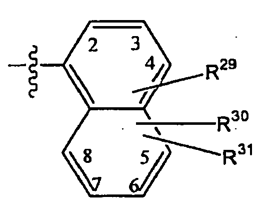 steht;
- Aryl³: für 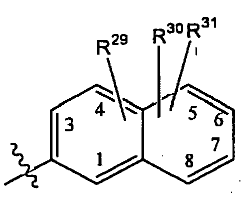 steht;
- R²⁹, R³⁰ und R³¹: unabhängig voneinander H, C₁₋₆-[Alk], C₃₋₈-[Cycloalk],-(C₁₋₆-Alkyl)-C₃₋₈-[Cycloalk], [Ar], -(C₁₋₆-Alkyl)-[Ar], [Het],-(C₁₋₆-Alkyl)-[Het], F, Cl, Br, I, -CN, -NC, -OR³², -SR³³,-NO, -NO₂, NH₂, NHR³⁴, NR³⁵R³⁶, -N-OH, -N-OC₁₋₆-[Alk], -NHNH₂, -N=N-[Ar], -(C=O)R³⁷, 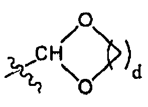 mit d = 1, 2 oder 3, oder -(C=S)R³⁷ bedeuten und in jeder beliebigen Ringposition sein können;
- R³² und R³³: unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
- R³⁴: C₁₋₆-[Alk], -CH₂-[Ar] oder -(C=O)O-tert.-Butyl bedeutet;
- R³⁵ und R³⁶: unabhängig voneinander C₁₋₆-[Alk] bedeuten oder gemeinsam für -(CH₂)_{g}- mit g = 4 oder 5 stehen;
- R³⁷: H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
- R³⁸: H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar] bedeutet;
und
- R³⁹: H, C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H, - CO₂-Alkyl,
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH; -CO₂H, - CO₂-Alkyl,
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrroldinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht,
wobei die Verbindungen
3,4-Dihydro-2-(4-nitrophenyl)-4-phenyl-2H-pyrimido[2,1-b]benzothiazol,
3,4-Dihydro-4-(4-methylphenyl)-2-(4-nitrophenyl)-2H-pyrimido[2,1-b]benzothiazol
ausgenommen sind.

Die erfindungsgemäßen Verbindungen der allgemeinen Struktur (II) in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate; in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; vorliegen. Die erfindungsgemäßen Verbindungen können in tautomeren Formen vorliegen, wobei die ggf. bevorzugte tautomere Form von Verbindung zu Verbindung und z.B. in Abhängigkeit vom Aggregatzustand oder vom gewählten Lösungsmittel variieren kann.

Folgende Verbindungen der allgemeinen Struktur (11) sind im Stand der Technik bereits bekannt, ohne daß deren Verwendung in einem Arzneimittel oder zur Herstellung eines Arzneimittels zur Therapie und/oder Prophylaxe von Schmerz, Epilepsie, Schizophrenie, neurodegenerativen Erkrankungen, insbesondere Morbus Alzheimer, Morbus Huntington und Morbus Parkinson, cerebralen Ischämien und Infarkten, Psychosen bedingt durch erhöhten Aminosäurespiegel, Hirnödemen, Unterversorgungszuständen des zentralen Nervensystems, insbesondere bei Hypoxien und Anoxien, der AIDS-Demens, der Encephalomyelitis, des Tourette-Syndroms, der perinatalen Asphyxie und bei Tinnitus beschrieben wird:
3,4-Dihydro-2-(4-nitrophenyl)-4-phenyl-2H-pyrimido[2,1-b]benzothiazol, 3,4-Dihydro-4-(4-methylphenyl)-2-(4-nitrophenyl)-2H-pyrimido[2,1-blbenzothiazol (M. A. Abdel-Rahman et al., CA (1995) 796768 [Rev. Roum. Chim. (1995) 42, 165-172]).
Diese Verbindungen sind daher insoweit ebenfalls Gegenstand der vorliegenden Erfindung, als erfindungsgemäße Verfahren zu ihrer Herstellung bzw. Arzneimittel sowie ihre Verwendung zur Herstellung von Medikamenten zur Behandlung und/oder Prophylaxe von Schmerz, Epilepsie, Schizophrenie, neurodegenerativen Erkrankungen, insbesondere Morbus Alzheimer, Morbus Huntington und Morbus Parkinson, cerebralen Ischämien und Infarkten, Psychosen bedingt durch erhöhten Aminosäurespiegel, Hirnödemen, Unterversorgungszuständen des zentralen Nervensystems, insbesondere bei Hypoxien und Anoxien, der AIDS-Demens, der Encephalomyelitis, des Tourette-Syndroms, der perinatalen Asphyxie und bei Tinnitus sowie von weiteren, in dieser Offenbarung genannten Krankheitszuständen betroffen sind.

Die Begriffe "Alkyl", "C₁₋₆-[Alk]" bzw. "C₁₋₆-Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von C₁₋₆-Alkyl) 1 bis 6 (d.h. 1, 2, 3, 4, 5 oder 6) C-Atomen, d.h. C₁₋₆-Alkanyle, C₂₋₆-Alkenyle und C₂₋₆-Alkinyle. Dabei weisen "Alkenyle" mindestens eine C-C-Doppelbindung und "Alkinyle" mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sek-Butyl, tert-Butyl, n-Pentyl, iso-Pentyl, neo-Pentyl, n-Hexyl, 2-Hexyl, n-Heptyl, n-Octyl, n-Nonyl, n-Decyl, n-Dodecyl; Ethenyl (Vinyl), Ethinyl, Propenyl (-CH₂CH=CH₂, -CH=CH-CH₃, -C(=CH₂)-CH₃), Propinyl (-CH₂-C=CH, -C=C-CH₃), Butenyl, Butinyl, Pentenyl, Pentinyl, Hexenyl, Hexinyl, Octenyl und Octinyl umfaßt. "C₃₋₈-[Cycloalk]" " bedeutet im Sinne dieser Erfindung einen cyclischen gesättigten oder ungesättigten Kohlenwasserstoff-Rest mit 3, 4, 5, 6, 7 oder 8 C-Atomen, wobei der Rest unsubstituiert oder einfach substituiert oder mehrfach gleich oder verschieden substituiert und ggf. benzokondensiert sein kann. Beispielhaft steht Cycloalkyl für Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl. Für die Zwecke der vorliegenden Erfindung besonders bevorzugt sind Cyclopropyl, Cyclopropyl-2-carbonsäure, Cyclopropyl-2-carbonsäureethylester und Cyclohexyl.

Die Ausdrücke "(C₁₋₆-Alkyl)-C₃₋₈-[Cycloalk]", "(C₁₋₆-Alkyl)-[Het] " und "(C₁₋₆-Alkyl)-[Ar]" bedeuten für die Zwecke der vorliegenden Erfindung, daß der [Cycloalk]-, [Het]- bzw. [Ar]-Rest über eine C₁₋₆-Alkyl-Gruppe an die mit ihm substituierte Verbindung gebunden ist. Entsprechendes gilt für den Ausdruck "CH₂-C₃₋₈-[Cycloalk]".

Im Zusammenhang mit "[Alk]", "Alkanyl", "Alkenyl", "Alkinyl" und "[Cycloalk]" versteht man unter dem Begriff "substituiert" im Sinne dieser Erfindung die Substitution eines Wasserstoffatoms durch beispielsweise F, Cl, Br, I, -CN, -NC, NH₂, NO₂, SH, OH, CO₂H, CO₂-Alkyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Besonders bevorzugt für die Zwecke der vorliegenden Erfindung sind CF₃ und CH₂-CH₂-OH als substituiertes Alkyl sowie Cyclopropyl-2-carbonsäure und Cyclopropyl-2-carbonsäureethylester als substituiertes Cycloalkyl.

In Bezug auf "Heterocyclyl" versteht man im Sinne dieser Erfindung unter "einfach substituiert" oder "mehrfach substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Heterocyclyl" nicht an anderer Stelle der Beschreibung oder in den Ansprüchen definiert ist, sind als geeignete Substituenten F, Cl, Br, I, -CN, NO₂, SH, OH, CO₂H, CO₂-Alkyl an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

"Benzokondensiert" bedeutet für die Zwecke der vorliegenden Erfindung, daß ein Benzol-Ring an einen anderen Cyclus ankondensiert ist.

Pharmazeutisch annehmbare bzw, physiologisch verträgliche Salze im Sinne dieser Erfindung sind solche Salze der erfindungsgemäßen Verbindungen gemäß der allgemeinen Struktur (II), die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren oder für den Fall, daß die erfindungsgemäßen Verbindungen Carbonsäuren sind, mit Basen gebildet werden.

Vorzugsweise werden die pharmazeutisch annehmbaren Salze der erfindungsgemäßen Verbindungen gemäß der allgemeinen Struktur (II) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Carbonsäuren, können die pharmazeutisch annehmbaren Salze auch durch Umsetzung mit Basen, wie z.B. Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um NatriumSalze. Besonders bevorzugt sind die Hydrochloride. Ebenfalls bevorzugt sind die Hydrate der erfindungsgemäßen Verbindungen, die z.B. durch Kristallisation aus wäßriger Lösung erhalten werden können.

Alle erfindungsgemäßen Verbindungen enthalten mindestens ein Asymmetriezentrum, nämlich das mit R⁵ substituierte Kohlenstoffatom der Struktur (II). Daher können die erfindungsgemäßen Verbindungen der allgemeinen Struktur (II) in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Bevorzugt liegen die Verbindungen der allgemeinen Struktur (II) als enantiomerenreine Verbindungen vor.

Unter diesen Verbindungen sind solche insbesondere bevorzugt, bei denen
- R¹ und R²: unabhängig voneinander H, O-R⁹, S-R¹⁰, unsubstituiertes oder einfach substituiertes oder mehrfach gleich oder verschieden substituiertes Methyl, Ethyl, n-Propyl, 2-Propyl, n-Butyl, tert.-Butyl oder n-Hexyl, wobei die Substituenten ausgewählt sind aus der Gruppe bestehend aus F, Cl, Br, I, -CN, -NC,-NH₂, -NO₂, -SH und -OH; Aryl' oder -CH₂-Aryl', wobei die Aryl'-Substituenten R²⁹, R³⁰ und R³¹ unabhängig voneinander H, Methyl, Ethyl, 2-Propyl, n-Butyl, tert.-Butyl, n-Hexyl, F, Cl, Br, I, OH, O-Methyl, O-Ethyl sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder Methyl, Ethyl, n-Propyl, 2-Propyl, n-Butyl, tert.-Butyl oder n-Hexyl bedeutet,
- R³ und R⁴: H, Methyl oder Aryl¹ bedeutet, wobei die Aryl¹-Substituenten R²⁹, R³⁰ und R³¹ unabhängig voneinander H, Methyl oder O-Methyl sind,
wobei mindestens einer der Reste R³ und R⁴ H ist, oder
- einer der Reste R¹ und R²: zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 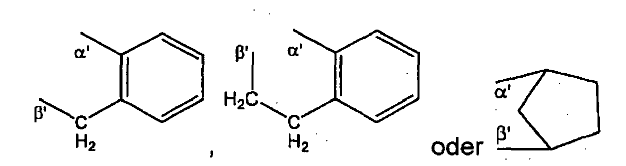 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² und der andere Rest von R³ und R⁴ jeweils H bedeuten;
- R⁵: Methyl, Ethyl, n-Propyl, 2-Propyl, n-Butyl, iso-Butyl, sek.-Butyl, tert.-Butyl, -(CH₂)₄-OH, Cyclopropyl, wobei Cyclopropyl unsubstituiert oder einfach mit C(=O)OH, C(=O)O-Methyl oder C(=O)O-Ethyl substituiert ist, Cyclopentyl, Cyclohexyl, Aryl¹ oder -(CH₂)ₖ-Aryl¹, wobei die Aryl¹-Substituenten R²⁹, R³⁰ und R³¹ unabhängig voneinander H, -OH, -O-Methyl, O-C₆H₅, CH₃, CF₃ oder C(=O)OH sind und k = 1 oder 2 ist, Heterocyclyl oder C(=O)R¹¹ bedeutet;
- R⁷: H, Aryl¹ mit R²⁹, R³⁰ und R³¹ gleich H, OH, S(O)_{q}R¹⁹,
wobei q = 0 oder 2 ist, oder Methyl, Ethyl, n-Propyl, 2-Propyl, n-Butyl, iso-Butyl, sek.-Butyl oder tert.-Butyl bedeutet,
- R⁸: H, Aryl¹, wobei die Aryl¹-Substituenten R²⁹ , R³⁰ und R³¹ unabhängig voneinander H, Methyl oder Chlor sind, oder Aryl³ mit R²⁹, R³⁰ und R³¹ gleich H bedeutet, oder
- die Reste R⁷ und R⁸: zusammen Y bilden, wobei Y γ'-CR²¹=CR²²-CR²³=CR²⁴-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist;
- R⁹: Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclopentyl oder Cyclohexyl bedeutet oder -[(CH₂)ᵣ-O]ₛ-H mit r = 1, 2 oder 3 und s = 1 oder 2 bedeutet;
- R¹⁰: Aryl¹ bedeutet;
- R¹¹: Aryl¹ mit R²⁹, R³⁰ und R³¹ gleich H oder OR²⁵ bedeutet;
- R¹⁹: Methyl oder Aryl¹ bedeutet, wobei einer der Aryl¹-Substiuenten R²⁹, R³⁰ und R³¹ gleich H oder -NO₂ ist und die beiden anderen Aryl¹-Substituenten von R²⁹, R³⁰ und R³¹ H sind,
- R²¹ und R²³: H bedeuten;
- R²²: H, Fluor oder OR²⁸ bedeutet;
- R²⁴: H oder Chlor bedeutet;
- R²⁵: H, Methyl oder Ethyl bedeutet, wobei R²⁵ nicht H bedeutet, wenn zugleich R¹ Aryl und R² Alkyl bedeuten;
- R²⁸: Methyl oder Ethyl bedeutet; und
- Heterocyclyl: Furan-2-yl, Furan-3-yl, Thien-2-yl, Thien-3-yl, Pyridin-2-y-, Pyridin-3-yl oder Pyridin-4-yl bedeutet, wobei Furanyl, Thienyl und Pyridinyl jeweils unsubstituiert oder einfach substituiert oder mehrfach gleich oder verschieden mit -NO₂, -CH₃ oder C(=O)OH substituiert sind.

Ganz besonders bevorzugte erfindungsgemäße Verbindungen der allgemeinen Struktur (II) sind solche, in denen
- R¹ und R²: unabhängig voneinander H, O-CH₂-CH₂-OH, O-Cyclohexyl, S-Phenyl, Methyl, Phenyl, 3-Fluor-phenyl, 3-Brom-phenyl, 4-Brom-phenyl, 4-Chlor-phenyl, 4-Fluor-phenyl, 3-Methyl-phenyl, 4-Hydroxy-phenyl, 4-Methoxy-phenyl, 2,4-Dimethyl-phenyl, 3,4-Dimethoxyphenyl, 2,3,4-Trimethoxyphenyl, 2-Naphthyl oder -CH₂-Phenyl bedeuten,
- R³ und R⁴: H, Methyl oder 4-Methoxy-phenyl bedeuten, wobei mindestens einer der Reste R³ und R⁴ H ist,
oder
- einer der Reste R¹ und R²: zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 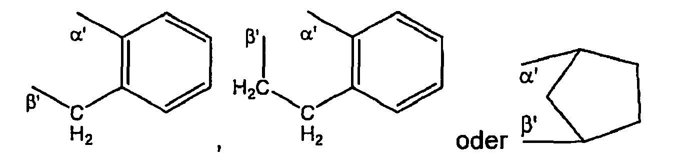 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² und der andere Rest von R³ und R⁴ jeweils H bedeuten;
- R⁵: n-Propyl, n-Butyl, tert.-Butyl, -(CH₂)₄-OH, Cyclopropyl, Cycloprop-2-yl-l-carbonsäureethylester, Cyclohexyl, 4-Trifluorphenyl, 4-Phenoxy-phenyl, 2-Hydroxy-3-methoxy-phenyl, 4-Hydroxy-3-methoxy-phenyl, 3-Carboxy-2-hydroxy-phenyl, -(CH₂)₂-Phenyl, 5-Carboxyfuran-2-yl, 5-Methyl-furan-2-yl, 5-Nitro-furan-2-yl, 5-Nitro-thien-2-yl, Pyridin-2-yl, Pyridin-3-yl, C(=O)Phenyl, C(=O)OH oder C(=O)OEthyl bedeutet, wobei R⁵ nicht C(=O)OH bedeutet, wenn zugleich R¹ Aryl und R² Alkyl bedeuten;
- R⁷: H, Phenyl, OH, -S-Methyl, -SO₂-(4-nitrophenyl) oder tert.-Butyl bedeutet,
- R⁸: 4-Chlor-phenyl, 4-Methyl-phenyl oder 2-Naphthyl bedeutet,
oder
- die Reste R⁷ und R⁸: zusammen Y bilden, wobei Y γ'-CR²¹=CH-CH=CH-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist; und
- R²¹: Fluor, Methoxy oder Ethoxy bedeutet.

Beispielhafte und vorteilhafte Verbindungen der vorliegenden Erfindung sind aus der Gruppe ausgewählt, die
- 2-(4-Nitro-phenylsulfonyl)-5-phenylsulfanyl-7-pyridin-2-yl-6,7-dihydro-5H-thiazolo[3,2-a]pyrimidin
- 3-(4-Chlor-phenyl)-5-phenylsulfanyl-7-pyridin-2-yl-6,7-dihydro-5H-thiazolo[3,2-a]pyrimidin
- 5-Phenylsulfanyl-7-pyridin-2-yl-3-p-tolyl-6,7-dihydro-5H-thiazolo[3,2-a]pyrimidin
- 7-Methoxy-4-phenylsulfanyl-2-pyridin-2-yl-3,4-dihydro-2H-9-thia-1,4a-diaza-fluoren
- 7-Ethoxy-4-phenylsulfanyl-2-pyridin-2-yl-3,4-dihydro-2H-9-thia-1,4a-diaza-fluoren
- 7-Fluor-4-phenylsulfanyl-2-pyridin-2-yl-3,4-dihydro-2H-9-thia-1,4a-diaza-fluoren
- 3-Naphthalin-2-yl-5-phenylsulfanyl-7-pyridin-2-yl-6,7-dihydro-5H-thiazolo[3,2-a]pyrimidin
umfaßt sowie ihre pharmazeutisch annehmbaren Salze.

Die Erfindung betrifft auch Verfahren zur Herstellung der erfindungsgemäßen Verbindungen der Struktur (II).

Entsprechend sind die erfindungsgemäßen Verbindungen der allgemeinen Struktur (II) sowie ihre pharmazeutisch annehmbaren Salze 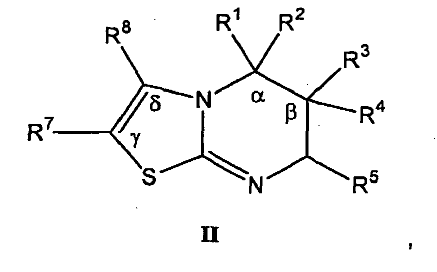 worin R¹, R², R³, R⁴, R⁵, R⁷ und R⁸ wie oben definiert sind, dadurch zugänglich, daß ein Thiazolamin der allgemeinen Struktur (VI) 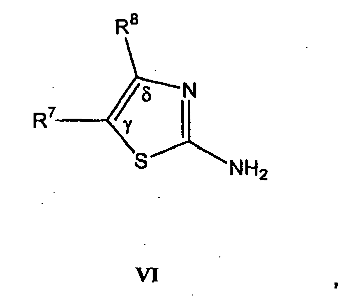 worin R⁷ und R⁸ wie oben definiert sind mit der Maßgabe, daß, wenn R⁷ und R⁸ Y bilden, das mit γ' gekennzeichnete Ende von Y mit dem mit γ gekennzeichneten Atom des Thiazolamins der allgemeinen Struktur (V1) und das mit δ' gekennzeichnete Ende von Y mit dem mit δ gekennzeichneten Atom des Thiazolamins der allgemeinen Struktur (V1) verknüpft sind, mit einem Aldehyd der allgemeinen Struktur (IV) 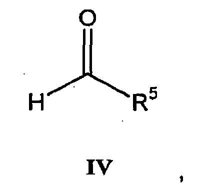 worin R⁵ wie oben definiert ist, und einem Olefin der allgemeinen Struktur (V) 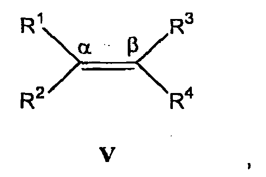 worin R¹, R², R³ und R⁴ wie oben definiert sind mit der Maßgabe, daß, wenn einer der Reste R¹ und R² zusammen mit einem der Reste R³ und R⁴ W bildet, das mit α' gekennzeichnete Ende von W mit dem α-Kohlenstoffatom des Olefins der allgemeinen Struktur (V) und das mit β' gekennzeichnete Ende von W mit dem β-Kohlenstoffatom des Olefins der allgemeinen Struktur (V) verbunden ist, in Gegenwart einer Säure umgesetzt wird.

Die erfindungsgemäßen Verfahren werden bevorzugt in einer "Eiritopf"-Reaktion durchgeführt, bei der je ein Heterocyclylamin der allgemeinen Struktur (VI), je ein Aldehyd der allgemeinen Struktur (IV) und je ein Olefin der allgemeinen Struktur (V) gleichzeitig miteinander umgesetzt werden.

Bei der eingesetzten Säure handelt es sich um eine anorganische oder organische Protonen- oder Lewis-Säure. Bevorzugt wird die Reaktion in Gegenwart einer organischen Säure, z.B. Essigsäure, Trifluoressigsäure oder Methansulfonsäure, insbesondere Trifluoressigsäure, durchgeführt.

Das erfindungsgemäße Herstellungsverfahren kann in jedem geeigneten Lösungsmittel durchgeführt werden, in dem die Reaktanten sich ausreichend lösen. Bevorzugt sind als Lösungsmittel organische Solventien, z.B. Dichlormethan oder insbesondere Acetonitril.

Die erfindungsgemäßen Verfahren werden zweckmäßig bei einer Temperatur von 0 bis 100°C, insbesondere bei 15 bis 40°C durchgeführt. Die Reaktionszeit beträgt vorzugsweise 15 Minuten bis 12 Stunden und kann den jeweiligen Erfordernissen angepaßt werden.

Alle in den erfindungsgemäßen Verfahren eingesetzten Heterocyclylamine der allgemeinen Struktur (VI), die Aldehyde der allgemeinen Struktur (IV) und die Olefine der allgemeinen Struktur (V) sind käuflich erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan) oder können nach im Stand der Technik allgemein bekannten Verfahren hergestellt werden.

Die erfindungsgemäßen Verfahren können auch in semi- oder vollautomatisierter Form als Parallelsynthese einer Gruppe von erfindungsgemäßen Verbindungen der allgemeinen Struktur (II) durchgeführt werden. Somit sind auch Substanzbibliotheken zugänglich, die mindestens eine Verbindung und vorzugsweise mindestens 48, insbesondere 96 und ganz besonders bevorzugt 384 Verbindungen der allgemeinen und wie oben definierten Struktur (II) enthalten.

Unter einer "Substanzbibliothek" wird dabei eine Gruppe von Verbindungen verstanden, die nach dem gleichen Verfahren unter gleichen oder nahezu gleichen Reaktionsbedingungen und unter Variation eines Reagenzes oder mehrerer Reagenzien hergestellt werden. Eine solche Substanzbibliothek kann die Bibliotheksmitglieder sowohl als einzelne reine Verbindungen als auch als Mischung dieser Verbindungen enthalten. Mit Hilfe dieser Substanzbibliothek kann beispielsweise ein medizinisches Screening in einem oder mehreren in-vitro-Screening-Verfahren in automatisierter Form durchgeführt werden.

Die Verbindungen der allgemeinen Struktur (II) können sowohl in Substanz als auch als Salz isoliert werden. Die Substanzen der allgemeinen Struktur (II) werden üblicherweise nach Umsetzung gemäß dem oben dargelegten Verfahren und anschließender herkömmlicher Aufarbeitung erhalten. Die so gewonnenen Verbindungen können dann beispielsweise durch Versetzen mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Soweit es sich bei den Verbindungen der allgemeinen Formel (II) um Säuren, insbesondere Carbonsäuren (z.B. wenn R⁵ = CO₂H), handelt, kann die Salzbildung durch Zugabe einer Base, z.B. Natriumhydroxid, NaHCO₃ oder Natriumcarbonat, herbeigeführt werden; für die (Carbon-)Säuren ist insbesondere die Bildung des Natriumsalzes bevorzugt. Die besonders bevorzugte Hydrochloridbildung kann insbesondere auch durch Versetzen der in einem geeigneten organischen Lösungsmittel gelösten Base (II) mit Trimethylsilylchlorid (TMSCI) herbeigeführt werden. Die Bildung von Natriumsalzen kann z.B. durch Titrieren der in einem geeigenten Lösungsmittel, beispielsweise Wasser-Methanol-Mischung, gelösten Verbindung (II) mit Natriumhydroxid-Lösung erfolgen.

Soweit die Verbindungen der allgemeinen Struktur (II) in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, oder- sofern es sich um Säuren handelt - mit chiralen Basen, etwa Brucin oder (-)-Ephedrin, gebildeten diastereomeren Salzen voneinander getrennt werden.

Darüber hinaus ist ein Arzneimittel, das mindestens eine der erfindungsgemäßen und wie oben definierten Verbindungen der allgemeinen Struktur (II) bzw. ihrer pharmazeutisch annehmbaren Salze umfaßt, Gegenstand der Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid oder ein Natriumsalz.

Ein weiterer Gegenstand der Erfindung ist die Verwendung mindestens einer erfindungsgemäßen Verbindung der allgemeinen Struktur (II), einschließlich ihrer Diastereomeren oder Enantiomeren, auch als Racemate oder Enantiomerengemisch, in Form ihrer freien Base oder Säure oder eines mit einer physiologisch verträglichen Säure bzw. Base gebildeten Salzes, insbesondere des Hydrochloridsalzes und des Natriumsalzes, zur Herstellung eines Arzneimittels zur Behandlung von Schmerz. Die erfindungsgemäßen Verbindungen haben sich als analgetisch wirksam erwiesen und binden an die MK801-Bindungsstelle des ionotropen NMDA-Rezeptors.

Es hat sich aufgrund der Bindung an die MK801-Bindungsstelle auch herausgestellt, daß die erfindungsgemäßen Verbindungen der allgemeinen Struktur (II) für weitere Indikationen, insbesondere zur Behandlung von Epilepsie, der Schizophrenie, von neurodegenerativen Erkrankungen, insbesondere Morbus Alzheimer, Morbus Huntington und Morbus Parkinson, von cerebralen Ischämien und Infarkten, Psychosen bedingt durch erhöhten Aminosäurespiegel, Hirnödemen, Unterversorgungszuständen des zentralen Nervensystems, insbesondere bei Hypoxien und Anoxien, von AIDS-Demens, von Encephalomyelitis, des Tourette-Syndroms, der perinatalen Asphyxie und bei Tinnitus, sehr geeignet sind. Ein weiterer Gegenstand der Anmeldung ist daher die Verwendung mindestens einer erfindungsgemäßen Verbindung der allgemeinen Struktur (II) einschließlich eines pharmazeutisch annehmbaren Salzes zur Herstellung eines Arzneimittels zur Behandlung und/oder Prophylaxe von Epilepsie, der Schizophrenie, von neurodegenerativen Erkrankungen, insbesondere Morbus Alzheimer, Morbus Huntington und Morbus Parkinson, von cerebralen Ischämien und Infarkten, Psychosen bedingt durch erhöhten Aminosäurespiegel, Hirnödemen, Unterversorgungszuständen des zentralen Nervensystems, insbesondere bei Hypoxien und Anoxien, von AIDS-Demens, von Encephalomyelitis, des Tourette-Syndroms, der perinatalen Asphyxie und/oder bei Tinnitus,

Darüber hinaus sind auch pharmazeutische Zusammensetzungen Gegenstand der vorliegenden Erfindung, die mindestens eine Verbindung der wie oben definierten allgemeinen Struktur (II) oder eines ihrer pharmazeutisch annehmbaren Salze und einen oder mehrere pharmazeutische Hilfsstoffe enthalten.

Die erfindungsgemäßen Arzneimittel und pharmazeutischen. Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermale therapeutische Systeme, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur (II) je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, vaginal, pulmonal, intraperitoneal, transdermal, intramuskulär, nasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Struktur (II) in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Struktur (II) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur (II) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur (II) appliziert.

Die nachfolgenden Beispiele dienen zur näheren Erläuterung der vorliegenden Erfindung:

### Beispiele

Die eingesetzten Chemikalien und Lösungsmittel wurden kommerziell von einem der folgenden Anbieter erworben: Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan; oder nach allgemeinen im Stand der Technik bekannten Verfahren hergestellt.

### Allgemeine Arbeitsvorschrift AAV (Semiautomatisierte Synthese)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt. Anschließend wurden nacheinander die folgenden Reagenzien hinzupipettiert:
1. 1 ml einer Lösung, die Trifluoressigsäure und die Heterocyclylamin-Komponente (III) bzw. (VI) jeweils 0, 1 M enthält, in Acetonitril
2. 1 ml einer 0,11 M Aldehyd(IV)-Lösung in Acetonitril
3. 1 ml einer 0,3 M Otefin(V)-Lösung in Acetonitril

Das Reaktionsgemisch wurde bei 20°C in einem der Rührblöcke 600 min lang gerührt. Danach wurde die Reaktionslösung an der Filtrations-Station abfiltriert. Das Röhrchen wurde dabei zweimal mit 1,5 ml einer 7,5% NaHCO₃-Lösung gespült. Das Rack mit den Proben wurde manuell auf die Aufarbeitungsanlage gestellt. Das Reaktionsgemisch wurde auf einem Vortexer mit 2 ml Diethylether versetzt und geschüttelt. Zur Ausbildung der Phasengrenze wurde in der Zentrifuge kurz zentrifugiert. Die Phasengrenze wurde optisch detektiert und die organische Phase abpipettiert.

Im nächsten Schritt wurde die wäßrige Phase erneut mit 2 ml Diethylether versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

Jede Probe wurde mit ESI-MS und/oder NMR analysiert. Massenspektrometrische Untersuchungen (ESI-MS) wurden mit einem Massenspektrometer der Fa. Finnegan, LCQ Classic durchgeführt. ¹H-NMR-Untersuchungen der erfindungsgemäßen Verbindungen wurden mit einem 300 MHz DPX Advance NMR-Gerät der Fa. Bruker durchgeführt.

Nach der angegebenen AAV wurden die Referenz-Verbindungen 1-132, 140-144, 146-151 sowie 153 und 154 sowie die Beispielverbindungen 133-139 (s. Tabelle 1) hergestellt.

**Tabelle 1**

| **Beispiel** | **Name** | **berechnete Masse** | **gefundene Masse** |
|---|---|---|---|
| 1 | 3-Brom-5-(5-nitro-furan-2-yl)-7-m-tolyl-tetrahydro-pyrazolo[1,5-a]pyrimidin | 403.24 | 403,2/405,1 |
| 2 | 3-Brom-7-(4-fluor-phenyl)-7-methyl-5-(5-nitro-furan-2-yl)-tetrahydro-pyrazolo[1,5-a]pyrimidin | 421.23 | 421,1/423,0 |
| 3 | 3-Brom-7-naphthalin-2-yl-5-(5-nitro-furan-2-yl)-tetrahydro-pyrazolo[1,5-a]pyrimidin | 439.27 | 439,2/441,1 |
| 4 | 2-(3-Brom-7-m-tolyl-tetrahydro-pyrazolo[1,5-a]pyrimidin-5-yl)-cyclopropancarbonsäureethylester | 404.31 | 404,5/406,4 |
| 5 | 2-[3-Brom-7-(4-brom-phenyl)-tetrahydro-pyrazolo[1,5-a]pyrimidin-5-yl]-cyclopropancarbonsäureethylester | 469.18 | 468,3/470,1/4 72,1 |
| 6 | 2-(3-Brom-7-naphthalin-2-yl-tetrahydro-pyrazolo[1,5-a]pyrimidin-5-yl)-cyclopropancarbonsaureethylester | 440.34 | 440,5/442,5 |
| 7 | 3-Brom-7-(4-fluor-phenyl)-7-methyl-5-(5-methyl-furan-2-yl)-tetrahydro-pyrazolo[1,5-a]pyrimidin | 390.26 | 390,1/392,0 |
| 8 | 3-Brom-7-(3,4-dimethoxy-phenyl)-tetrahydro-pyrazolo[1,5-a]pyrimidin-5-carbonsäureethylester | 410.27 | 410,3/412,2 |
| 9 | 3-Brom-7-(4-methoxy-phenyl)-tetrahydro-pyrazolo[1,5-a]pyrimidin-5-carbonsäureethylester | 380.24 | 380,2/382,1 |
| 10 | 3-Brom-7-(4-methoxy-phenyl)-tetrahydro-pyrazolo[1,5-a]pyrimidin-5-carbonsäure | 352.19 | 354,2 |
| 11 | 3-Brom-7-(2,4-dimethyl-phenyl)-5-(5-nitro-furan-2-yl)-tetrahydro-pyrazolo[1,5-a]pyrimidin | 417.26 | 417,1/419,0 |
| 12 | 3-Brom-7-(4-methoxy-phenyl)-5-(5-nitro-furan-2-yl)-tetrahydro-pyrazolo[1,5-a]pyrimidin | 419.23 | 419,0/421,0 |
| 13 | 5,5a,6,8a-Tetrahydro-3H-1,4,8b-triaza-as-indacen-3,5-dicarbonsäurediethylester; 5,5a,6,8a-Tetrahydro-4H-1,4,8b-triaza-as-indacen-3,5-dicarbonsäurediethylester; | 305.33 | 306,1 |
| 14 | 2-Hydroxy-3-phenylazo-5,5a,6,8a-tetrahydro-3H-1,4,8b-triaza-as-indacen-5-carbonsäureethylester, 2-Hydroxy-3-phenylazo-5,5a,6,8a-tetrahydro-4H-1,4,8b-triaza-as-indacen-5-carbonsäureethylester | 353.38 | 354,3 |
| 15 | *2-tert-*Butyl-5,5a,6,8a-tetrahydro-3H-1,4,8b-triaza-as-indacen-5-carbonsäureethylester; 2-tert-Butyl-5,5a,6,8a-tetrahydro-3H-1,4,8b-triaza-as-indacen-5-carbonsäureethylester | 289.37 | 290,3 |
| 16 | 3-Brom-2-phenyl-5,5a,6,8a-tetrahydro-3H-1,4,8b-triaza-as-indacen-5-carbonsäureethylester; 3-Brom-2-phenyl-5,5a,6,8a-tetrahydro-4H-1,4,8b-triaza-as-indacen-5-carbonsäureethylester | 388.26 | 388,2/390,1 |
| 17 | 7-(2,3,4-Trimethoxy-phenyl)-tetrahydro-pyrazolo[1,5-a]pyrimidin-3,5-dicarbonsäurediethyl ester | 433.46 | 434,4 |
| 18 | 3-Cyano-2-methylsulfanyl-7-(2,3,4-trimethoxy-phenyl)-tetrahydro-pyrazolo[1,5-a]pyrimidin-5-carbonsäureethylester | 432.49 | 433,2 |
| 19 | 2-Hydroxy-7-(4-hydroxy-phenyl)-6-methyl-3-phenylazo-fetrahydro-pyrazolo[1,5-a]pyrimidin-5-carbonsäureethylester | 421.45 | 422,4 |
| 20 | 3-Brom-7-(4-hydroxy-phenyl)-6-methyl-2-phenyl-tetrahydro-pyrazolo[1,5-a]pyrimidin-5-carbonsäureethylester | 456.34 | 456,4/458,4 |
| 21 | 5,5a,6,10b-Tetrahydro-3H-1,4,10c-triaza-cyclopenta[c]fluoren-3,5-dicarbonsäurediethylester; 5,5a,6,10b-Tetrahydro-4H-1,4,10c-triaza-cyclopenta[c]fluoren-3,5-dicarbonsäurediethylester | 355.39 | 356,2 |
| 22 | 2-Hydroxy-3-phenylazo-5,5a,6,10b-tetrahydro-3H-1,4,10c-triaza-cyclopenta[c]fluoren-5-carbonsäureethylester; 2-Hydroxy-3-phenylazo-5.5a,6,10b-tetrahydro-4H-1,4.10c-triaza-cyclopenta[c]fluoren-5-carbonsäureethylester | 403.44 | 404,3 |
| 23 | 7-Phenylsulfanyl-tetrahydro-pyrazolo[1,5-a]pyrimidin-3,5-dicarbonsäurediethyl ester | 375.44 | 376,2 |
| 24 | 3-Cyano-2-methylsulfanyl-7-phenylsulfanyl-tetrahydro-pyrazolo[1,5-a]pyrimidin-5-carbonsäureethylester | 374.48 | 375,1 |
| 25 | 3-Cyano-2-methylsulfanyl-7-(2,3,4-trimethoxy-phenyl)-tetrahydro-pyrazolo[1,5-a]pyrimidin-5-carbonsäure | 404.44 | 405,2 |
| 26 | 7-Phenylsulfanyl-tetrahydro-pyrazolo[1,5-a]pyrimidin-3,5-dicarbonsäure3-ethyl ester | 347.39 | 348,2 |
| 27 | 3-Cyano-7-(2,4-dimethyl-phenyl)-2-methylsulfanyl-tetrahydro-pyrazolo[1,5-a]pyrimidin-5-carbonsäureethylester | 370.47 | 371,2 |
| 28 | 3-Cyano-7-(2,4-dimethyl-phenyl)-tetrahydro-pyrazolo[1,5-a]pyrimidin-5-carbonsäureethylester | 324.38 | 325,2 |
| 29 | 7-(2,4-Dimethyl-phenyl)-tetrahydro-pyrazolo[1,5-alpyrimidin-3,5-dicarbonsäure-3-ethyl ester | 343.38 | 344,2 |
| 30 | 3-Brom-7-(2,4-dimethyl-phenyl)-2-phenyl-tetrahydro-pyrazolo[1,5-a]pyrimidin-5-carbonsäure | 426.31 | 426,2/428,1 |
| 31 | 3-Cyano-7-(2,4-dimethyl-phenyl)-2-methylsulfanyl-tetrahydro-pyrazolo[1,5-a]pyrimidin-5-carbonsäure | 342.42 | 343,2 |
| 32 | 3-Cyano-7-(2,4-dimethyl-phenyl)-tetrahydro-pyrazolo[1,5-a]pyrimidin-5-carbonsäure | 296.32 | 297,2 |
| 33 | 3-Cyano-7-(3,4-dimethoxy-phenyl)-2-methylsulfanyl-tetrahydro-pyrazolo[1,5-a]pyrimidin-5-carbonsäure | 374.41 | 376,2 |
| 34 | 7-(2,4-Dimethyl-phenyl)-5-(5-nitro-furan-2-yl)-tetrahydro-pyrazolo[1,5-a]pyrimidin-3-carbonsäureethylester | 410,42 | 411,1 |
| 35 | 7-(2,4-Dimethyl-phenyl)-5-(5-nitro-furan-2-yl)-3-phenylazo-tetrahydro-pyrazolo[1,5-a]pyrimidin-2-ol | 458.47 | 459,3 |
| 36 | 3-Brom-7-(2,4-dimethyl-phenyl)-5-(5-nitro-furan-2-yl)-2-phenyl-tetrahydro-pyrazolo[1,5-a]pyrimidin | 493.36 | 493,2/495,1 |
| 37 | 7-(2,4-Dimethyl-phenyl)-2-methylsulfanyl-5-(5-nitro-furan-2-yl)-tetrahydro-pyrazolo[1,5-a]pyrimidin-3-carbonitril | 409.46 | 410,1 |
| 38 | 7-(2,4-Dimethyl-phenyl)-5-(5-nitro-furan-2-yl)-tetrahydro-pyrazolo[1, 5-a]pyrimidin-3-carbonitril | 363.37 | 364,1 |
| 39 | 3-Brom-7-(3,4-dimethoxy-phenyl)-5-(5-nitro-furan-2-yl)-2-phenyl-tetrahydro-pyrazolo[1,5-a]pyrimidin | 525.36 | 525,4/527,1 |
| 40 | 7-(4-Methoxy-phenyl)-2-methylsulfanyl-5-(5-nitro-furan-2-yl)-tetrahydro-pyrazolo[1,5-a]pyrimidin-3-carbonitril | 411.43 | 412,9 |
| 41 | 7-(2,4-Dimethyl-phenyl)-5-(2-ethoxycarbonyl-cyclopropyl)-tetrahydro-pyrazolo[1,5-a]pyrimidin-3-carbonsäureethylester | 411.5 | 412,5 |
| 42 | 2-[7-(2,4-Dimethyl-phenyl)-2-hydroxy-3-phenylazo-tetrahydro-pyrazolo[1,5-a]pyrimidin-5-yl]-cyclopropancarbonsäureethylester | 459.54 | 460,3 |
| 43 | 2-[2-tert-Butyl-7-(2,4-dimethyl-phenyl)-tetrahydro-pyrazolo[1,5-a]pyrimidin-5-yl]-cyclopropancarbonsäureethylester | 395.54 | 396,5 |
| 44 | 2-[3-Brom-7-(2,4-dimethyl-phenyl)-2-phenyl-tetrahydro-pyrazolo[1,5-a]pyrimidin-5-yl]-cyclopropancarbonsäureethylester | 494.43 | 494,4/496,2 |
| 45 | 2-[3-Cyano-7-(2,4-dimethyl-phenyl)-2-methylsulfanyl-tetrahydro-pyrazolo[1,5-a]pyrimidin-5-yl]-cyclopropancarbonsäureethylester | 410.53 | 411,3 |
| 46 | 5-(2-Ethoxycarbonyl-cyclopropyl)-7-(3-fluor-phenyl)-tetrahydro-pyrazolo[1, 5-a]pyrimidin-3-carbonsäureethylester | 401.43 | 402,2 |
| 47 | 2-[3-Brom-7-(3-brom-phenyl)-2-phenyl-tetrahydro-pyrazolo[1,5-a]pyrimidin-5-yl]-cyclopropancarbonsäureethylester | 545.28 | 544,3/546,1/5 48,0 |
| 48 | 2-[7-(3-Brom-phenyl)-3-cyano-2-methylsulfanyl-tetrahydro-pyrazolo[1,5-a]pyrimidin-5-yl]-cyclopropancarbonsäureethylester | 461.38 | 461,21463,0 |
| 49 | 7-(2,4-Dimethyl-phenyl)-5-(5-nitro-thiophen-2-yl)-3-phenylazo-tetrahydro-pyrazolo[1,5-a]pyrimidin-2-ol | 474.54 . | 475,2 |
| 50 | 7-(2,4-Dimethyl-phenyl)-2-methylsulfanyl-5-(5-nitro-thiophen-2-yl)-tetrahydro-pyrazolo[1,5-a]pyrimidin-3-carbonitril | 425.53 | 426,1 |
| 51 | 7-(2,4-Dimethyl-phenyl)-5-(5-nitro-thiophen-2-yl)-tetrahydro-pyrazolo[1,5-a]pyrimidin-3-carbonitril | 379.44 | 380,1 |
| 52 | 7-(3,4-Dimethoxy-phenyl)-5-(5-nitro-thiophen-2-yl)-tetrahydro-pyrazolo[1,5-a]pyrimidin-3-carbonsäureethylester | 458.49 | 459,3 |
| 53 | 7-(3,4-Dimethoxy-phenyl)-5-(5-nitro-thiophen-2-yl)-3-phenyiazo-tetrahydro-pyrazolo[1,5-a]pyrirnidin-2-ol | 506.54 | 507,2 |
| 54 | 3-Brom-7-(3,4-dimethoxy-phenyl)-5-(5-nitro-thiophen-2-yl)-2-phenyl-tetrahydro-pyrazolo[1,5-a]pyrimidin | 541.42 | 541,4/543,3 |
| 55 | 7-(3,4-Dimethoxy-phenyl)-2-methylsulfanyl-5-(5-nitro-thiophen-2-yl)-tetrahydro-pyrazolo[1,5-a]pyrimidin-3-carbonitril | 457.53 | 458,1 |
| 56 | 7-(3,4-Dimethoxy-phenyl)-5-(5-nitro-thiophen-2-yl)-tetrahydro-pyrazolo[1,5-a]pyrimidin-3-carbonitril | 411.43 | 412,1 |
| 57 | 7-(4-Methoxy-phenyl)-5-(5-nitro-thiophen-2-yl)-tetrahydro-pyrazolo[1,5-a]pyrimidin-3-carbonsäureethylester | 428.46 | 429,1 |
| 58 | 5-[3-Brom-7-(4-methoxy-phenyl)-2-phenyl-tetrahydro-pyrazolo[1,5-a]pyrimidin-5-yl]-furan-2-carbonsaure | 494.34 | 494,2/496,1 |
| 59 | 5-Benzoyl-7-(2,4-dimethyl-phenyl)-tetrahydro-pyrazolo[1,5-a]pyrimidin-3-carbonsäureethylester | 403.48 | 404,2 |
| 60 | 5-Benzoyl-7-(2,4-dimethyl-phenyl)-2-methylsulfanyl-tetrahydro-pyrazolo[1,5-a]pyrimidin-3-carbonitril | 402.51 | 403,1 |
| 61 | 5-Benzoyl-7-(2,4-dimethyl-phenyl)-tetrahydro-pyrazolo[1,5-a]pyrimidin-3-carbonitril | 356.42 | 355,31357;2 |
| 62 | 5-Benzoyl-7-(3,4-dimethoxy-phenyl)-tetrahydro-pyrazolo[1,5-a]pyrimidin-3-carbonsäureethylester | 435.47 | 436,2 |
| 63 | [3-Brom-7-(3,4-dimethoxy-phenyl)-2-phenyl-tetrahydro-pyrazolo[1,5-a]pyrimidin-5-yl]-phenyl-methanon | 518.41 | 518,71520,2 |
| 64 | 5-Benzoyl-7-(3,4-dimethoxy-phenyl)-2-methylsulfanyl-tetrahydro-pyrazolo[1,5-a]pyrim idin-3-carbonitril | 434.51 | 435,1 |
| 65 | 5-Benzoyl-7-(3,4-dimethoxy-phenyl)-tetrahydro-pyrazolo[1,5-a]pyrimidin-3-carbonitril | 388.42 | 389,1 |
| 66 | 5-Benzoyl-7-(4-methoxy-phenyl)-tetrahydro-pyrazolo[1,5-a]pyrimidin-3-carbonsäureethylester | 405.45 | 406,1 |
| 67 | 5-Benzoyl-7-(4-methoxy-phenyl)-2-methylsulfanyl-tetrahydro-pyrazolo[1,5-a]pyrimidin-3-carbonitril | 404.49 | 405,0 |
| 68 | 5-Benzoyl-7-(4-methoxy-phenyl)-tetrahydro-pyrazolo[1,5-a]pyrimidin-3-carbonitril | 358.4 | 359,0 |
| 69 | 5-Benzoyl-7-(3-fluor-phenyl)-tetrahydro-pyrazolo[1,5-a]pyrimidin-3-carbonsäureethylester | 393.41 | 394,4 |
| 70 | [3-Brom-7-(3-fluor-phenyl)-2-phenyl-tetrahydro-pyrazolo[1,5-a]pyrimidin-5-yl]-phenyl-methanon | 476.35 | 476,3/478,3 |
| 71 | [3-Brom-7-(3-brom-phenyl)-2-phenyl-tetrahydro-pyrazolo[1,5-a]pyrimidin-5-yl]-phenyl-methanon | 537.26 | 538,4 |
| 72 | 7-(2,4-Dimethyl-phenyl)-5-(4-phenoxy-phenyl)-tetrahydro-pyrazolo[1,5-a]pyrimidin-3-carbonsäureethylester | 467.56 | 468,2 |
| 73 | 3-Brom-7-(2,4-dimethyl-phenyl)-5-(4-phenoxy-phenyl)-2-phenyl-tetrahydro-pyrazolo[1,5-a]pyrimidin | 550.5 | 550,3/552.2 |
| 74 | 7-(2,4-Dimethyl-phenyl)-2-methylsulfanyl-5-(4-phenoxy-phenyl)-tetrahydro-pyrazolo[1,5-a]pyrimidin-3-carbonitril | 466.6 | 467,1 |
| 75 | 7-(2,4-Dimethyl-phenyl)-5-(4-phenoxy-phenyl)-tetrahydro-pyrazolo[1,5-a]pyrimidin-3-carbonitril | 420.51 | 421,1 |
| 76 | 7-(3,4-Dimethoxy-phenyl)-5-(4-phenoxy-phenyl)-tetrahydro-pyrazolo[1,5-a]pyrimidin-3-carbonsäureethylester | 499.56 | 500,2 |
| 77 | 7-(3,4-Dimethoxy-phenyl)-2-methylsulfanyl-5-(4-phenoxy-phenyl)-tetrahydro-pyrazolo[1,5-a]pyrimidin-3-carbonitril | 498.6 | 499,1 |
| 78 | 3-[3-Cyano-7-(4-hydroxy-phenyl)-6-methyl-tetrahydro-pyrazolo[1,5-a]pyrimidin-5-yl]-2-hydroxy-benzoesäure | 390.39 | 391,1 |
| 79 | 3-(3-Cyano-5,5a,6,10b-tetrahydro-3H-1,4,10c-triaza-cyclopenta[c]fluoren-5-yl)-2-hydroxy-benzoesäure; 3-(3-Cyano-5,5a,6,10b-tetrahydro-4H-1,4,10c-triaza-cyclopenta[c]fluoren-5-yl)-2-hydroxy-benzoesäure | 372.38 | 373,0 |
| 80 | 3-(3-Cyano-7-phenylsulfanyl-tetrahydro-pyrazolo[1,5-a]pyrimidin-5-yl)-2-hydroxy-benzoesäure | 392.43 | 392,9 |
| 81 | 3-[2-tert-Butyl-7-(4-chlor-phenyl)-7-methyl-tetrahydro-pyrazolo[1,5-a]pyrimidin-5-yl]-2-hydroxy-benzoesäure | 439.94 | 440,2 |
| 82 | 5-(4-Hydroxy-3-methoxy-phenyl)-7-(4-hydroxy-phenyl)-6-methyl-tetrahydro-pyrazolo[1,5-a]pyrimidin-3-carbonsäureethylester | 423.46 | 424,1 |
| 83 | 5-(4-Hydroxy-3-methoxy-phenyl)-7-(4-hydroxy-phenyl)-6-methyl-tetrahydro-pyrazolo[1,5-a]pyrimidin-3-carbonitril | 376.41 | 377,1 |
| 84 | 5-(4-Hydroxy-3-methoxy-phenyl)-5,5a,6,10b-tetrahydro-3H-1,4,10c-triaza-cyclopenta[c]fluoren-3-carbonsäureethylester; 5-(4-Hydroxy-3-methoxy-phenyl)-5,5a,6,10b-tetrahydro-4H-1,4,10c-triaza-cyclopenta[c]fluoren-3-carbonsäureethylester | 405.45 | 406,0 |
| 85 | 4-(2-tert-Butyl-5,5a,6,10b-tetrahydro-3H-1,4,10c-triaza-cyciopenta[c]fluoren-5-yl)-2-methoxy-phenol; 4-(2-tert-Butyl-5,5a,6,10b-tetrahydro-4H-1,4,10c-triaza-cyclopenta[c]fluoren-5-yl)-2-methoxy-phenol | 389.49 | 390,2 |
| 86 | 5-(4-Hydroxy-3-methoxy-phenyl)-2-methylsulfanyl-5,5a,6,10b-tetrahydro-3H-1,4,10c-triaza-cyclopenta[c]fluoren-3-carbonitril; 5-(4-Hydroxy-3-methoxy-phenyl)-2-methylsulfanyl-5,5a,6,10b-tetrahydro-3H-1,4,10c-triaza-cyclopenta[c]fluoren- 3-carbonitril | 404.49 | 404,9 |
| 87 | 5-(4-Hydroxy-3-methoxy-phenyl)-7-phenylsulfanyl-tetrahydro-pyrazolo[1,5-a]pyrimidin-3-carbonsäureethylester | 425.5 | 426,0 |
| 88 | 4-(2-tert-Butyl-7-phenylsulfanyl-tetrahydro-pyrazolo[1,5-a]pyrimidin-5-yl)-2-methoxy-phenol | 409.55 | 410,1 |
| 89 | 4-(3-Brom-2-phenyl-7-phenylsulfanyl-tetrahydro-pyrazolo[1,5-a]pyrimidin-5-yl)-2-methoxy-phenol | 508.44 | 508,2/510,0 |
| 90 | 5-(2-Hydroxy-3-methoxy-phenyl)-7-phenylsulfanyl-tetrahydro-pyrazolo[1,5-a]pyrimidin-3-carbonsäureethylester | 425.5 | 425,0 |
| 91 | 7-(4-Chlor-phenyl)-5-(2-hydroxy-3-methoxy-phenyl)-7-methyl-tetrahydro-pyrazolo[1,5-a]pyrimidin-3-carbonsäureethylester | 441.91 | 441,0 |
| 92 | 5-(4-Hydroxy-butyl)-5,5a,6,10b-tetrahydro-3H-1,4,10c-triaza-cyclopenta[c]fluoren-3-carbonitril; 5-(4-Hydroxy-butyl)-5;5a,6,10b-tetrahydro-4H-1,4,10c-triaza-cyclopenta[c]fluoren-3-carbonitril | 308.38 | 309,3 |
| 93 | 5-(4-Hydroxy-butyl)-2-methylsulfanyl-7-phenylsulfanyl-tetrahydro-pyrazolo[1,5-a]pyrimidin-3-carbonitril | 374.52 | 375,2 |
| 94 | 5-(4-Hydroxy-butyl)-7-phenylsulfanyl-tetrahydro-pyrazolo[1,5-a]pyrimidin-3-carbonitril | 328.43 | 329,2 |
| 95 | 7-(4-Chlor-phenyl)-5-(4-hydroxy-butyl)-7-methyl-tetrahydro-pyrazolo[1,5-a]pyrimidin-3-carbonitril | 344.84 | 345,1 |
| 96 | 5-Butyl-2-methylsulfanyl-5,5a,6,10b-tetrahydro-3H-1,4,10c-triaza-cyclopenta[c]fluoren-3-carbonitril; 5-Butyl-2-methylsulfanyl-5,5a,6,10b-tetrahydro-4H-1,4,10c-triaza-cyclopenta[c]fluoren-3-carbonitril | 338.47 | 339,3 |
| 97 | 5-Butyl-2-methylsulfanyl-7-phenylsulfanyl-tetrahydro-pyrazolo[1,5-a]pyrimidin-3-carbonitril | 358.52 | 359,2 |
| 98 | 5-Butyl-7-phenylsulfanyl-tetrahydro-pyrazolo[1,5-a]pyrimidin-3-carbonitril | 312.43 | 313,1 |
| 99 | 5-Butyl-7-(4-chlor-phenyl)-7-methyl-2-methylsulfanyl-tetrahydro-pyrazolo[1,5-a]pyrimidin-3-carbonitril | 374.93 | 375,3 |
| 100 | 5-Cyclopropyl-7-(2,4-dimethyl-phenyl)-3-phenylazo-tetrahydro-pyrazolo[1,5-a]pyrimidin-2-ol | 387.48 | 388,5 |
| 101 | 2-tert-Butyl-5-cyclopropyl-7-(2,4-dimethyl-phenyl)-tetrahydro-pyrazolo[1,5-a]pyrimidin | 323.48 | 324,6 |
| 102 | 5-Cyclopropyl-7-(2,4-dimethyl-phenyl)-2-methylsulfanyl-tetrahydro-pyrazolo[1,5-a]pyrimidin-3-carbonitril | 338.47 | 339,8 |
| 103 | 2-tert-Butyl-5-cyclopropyl-7-(3,4-dimethoxy-phenyl)-tetrahydro-pyrazolo[1,5-a]pyrimidin | 355.48 | 356,3/357,6 |
| 104 | 3-Brom-5-cyclopropyl-7-(3,4-dimethoxy-phenyl)-2-phenyl-tetrahydro-pyrazolo[1,5-a]pyrimidin | 454.37 | 454,3/456,1 |
| 105 | 5-Cyclopropyl-7-(4-methoxy-phenyl)-tetrahydro-pyrazolo[1,5-a]pyrimidin-3-carbonsäureethylester | 341.41 | 342,7 |
| 106 | 5-Cyclopropyl-3,5,5a,6,7,11b-hexahydro-1,4,11c-triaza-cyclopenta[c]phenanthrene-3-carbonitril | 290.36 | 291,4 |
| 107 | 7-(2,4-Dimethyl-phenyl)-5-pyridin-2-yl-tetrahydro-pyrazolo[1,5-a]pyrimidin-3-carbonsäureethylester | 376.45.. | 377,3/378,4 |
| 108 | 7-(2,4-Dimethyl-phenyl)-3-phenylazo-5-pyridin-2-yl-tetrahydro-pyrazolo[1,5-a]pyrimidin-2-ol | 424.5 | 425,3 |
| 109 | 3-Brom-7-(2,4-dimethyl-phenyl)-2-phenyl-5-pyridin-2-yl-tetrahydro-pyrazolo[1,5-a]pyrimidin | 459.39 | 459,7/462,2 |
| 110 | 7-(2,4-Dimethyl-phenyl)-2-methylsulfanyl-5-pyridin-2-yl-tetrahydro-pyrazolo[1,5-a]pyrimidin-3-carbonitril | 375.49 | 376,4 |
| 111 | 7-(3,4-Dimethoxy-phenyl)-2-methylsulfanyl-5-phenethyl-tetrahydro-pyrazolo[1,5-a]pyrimidin-3-carbonitril | 434.56 | 435,3 |
| 112 | 7-(3,4-Dimethoxy-phenyl)-5-phenethyl-tetrahydro-pyrazolo[1,5-a]pyrimidin-3-carbonitril | 388.47 | 389,2 |
| 113 | 5-Cyclopropyl-7-(2-hydroxy-ethoxy)-tetrahydro-pyrazolo[1,5-a]pyrimidin-3-carbonsäureethylester | 295.33 | 296,2 |
| 114 | 2-(2-tert-Butyl-5-cyclopropyl-tetrahydro-pyrazolo[1,5-a]pyrimidin-7-yloxy)-ethanol | 279.38 | 280,3 |
| 115 | 5-Cyclopropyl-3,5,5a,6,7,8a-hexahydro-8-oxa-1,4,8b-triaza-as-indacen-3-carbonsäureethylester; 5-Cyclopropyl-4,5,5a,6,7,8a-hexahydro-8-oxa-1,4,8b-triaza-as-indacen-3-carbonsäureethylester | 277.32 | 278,3 |
| 116 | 5-Cyclopropyl-3-phenylazo-3,5,5a,6,7,8a-hexahydro-8-oxa-1,4,8b-triaza-as-indacen-2-ol; 5-Cyclopropyl-3-phenylazo-4,5,5a,6,7,8a-hexahydro-8-oxa-1,4,8b-triaza-as-indacen-2-ol | 325.37 | 326,5 |
| 117 | 7-Cyclohexyloxy-5-cyclopropyl-tetrahydro-pyrazolo[1,5-a]pyrimidin-3-carbonsäureethylester | 333.43 | 334,1 |
| 118 | 7-Cyclohexyloxy-5-cyclopropyl-2-methylsulfanyl-tetrahydro-pyrazolo[1,5-a]pyrimidin-3-carbonitril | 332.46 | 333,2 |
| 119 | 7-(4-Chlor-phenyl)-5-cyclohexyl-tetrahydro-pyrazolo[1,5-a]pyrimidin-3-carbonitril | 340.85 | 341,4 |
| 120 | 5-Cyclohexyl-7-(2-hydroxy-ethoxy)-tetrahydro-pyrazolo[1,5-a]pyrimidin-3-carbonsäureethylester | 337.41 | 338,3 |
| 121 | 5-Cyclohexyl-3,5,5a,6,7,8a-hexahydro-8-oxa-1,4,8b-triaza-as-indacen-3-carbonsäureethylester; 5-Cyclohexyl-4,5,5a,6,7,8a-hexahydro-8-oxa-1,4,8b-triaza-as-indacen-3-carbonsäureethylester | 319.4 | 320,3 |
| 122 | 5-Cyclohexyl-7-cyclohexyloxy-tetrahydro-pyrazolo[1,5-a]pyrimidin-3-carbonsäureethylester | 375.51 | 376,2 |
| 123 | 7-(2,4-Dimethyl-phenyl)-3-phenylazo-5-propyl-tetrahydro-pyrazolo[1,5-a]pyrimidin-2-ol | 389.5 | 390,5 |
| 124 | 7-(2,4-Dimethyl-phenyl)-2-methylsulfanyl-5-propyl-tetrahydro-pyrazolo[1,5-a]pyrimidin-3-carbonitril | 340.49 | 341,3 |
| 125 | 5-tert-Butyl-7-(2,4-dimethyl-phenyl)-tetrahydro-pyrazolo[1,5-a]pyrimidin-3-carbonsäureethylester | 355.48 | 356,1 |
| 126 | 2,5-Di-tert-butyl-7-(3,4-dimethoxy-phenyl)-tetrahydro-pyrazolo[1, 5-a]pyrimidin | 371.52 | 372,2 |
| 127 | 3-Brom-5-tert-butyl-7-(3,4-dimethoxy-phenyl)-2-phenyl-tetrahydro-pyrazolo[1,5-a]pyrimidin | 470.41 | 470,2/472,1 |
| 128 | 2-[3-Cyano-6,7-bis-(4-methoxy-phenyl)-tetrahydro-pyrazolo[1,5-a]pyrimidin-5-yl]-cyclopropancarbonsäureethylester | 472.54 | 473,0 |
| 129 | 3-Cyano-6,7-bis-(4-methoxy-phenyl)-tetrahydro-pyrazolo[1,5-a]pyrimidin-5-carbonsäure | 404.42 | 405,0 |
| 130 | 4-[3-Brom-6-methyl-2-phenyl-5-(4-trifluormethyl-phenyl)-tetrahydro-pyrazolo[1,5-a]pyrimldin-7-yl]-phenol | 528.37 | 528,1 |
| 131 | 7-(4-Hydroxy-phenyl)-6-methyl-2-methylsulfanyl-5-(4-trifluormethyl-phenyl)-tetrahydro-pyrazolo[1,5-a]pyrimidin-3-carbonitril | 444.47 | 445,1 |
| 132 | 7-(4-Hydroxy-phenyl)-6-methyl-5-(4-trifluormethyl-phenyl)-tetrahydro-pyrazolo[1, 5-a]pyrimidin-3-carbonitril | 398.38 | 399,1 |
| 133 | 2-(4-Nitro-phenylsulfonyl)-5-phenylsulfanyl-7-pyridin-2-yl-6,7-dihydro-5H-thiazolo[3,2-a]pyrimidin | 510.61 | 511,2 |
| 134 | 3-(4-Chlor-phenyl)-5-phenylsulfanyl-7-pyridin-2-yl-6,7-dihydro-5H-thiazolo[3,2-a]pyrimidin | 435.99 | 436,4 |
| 135 | 5-Phenylsulfanyl-7-pyridin-2-yl-3-p-tolyl-6,7-dihydro-5H-thiazolo[3,2-a]pyrimidin | 415.58 | 416,3 |
| 136 | 7-Methoxy-4-phenylsulfanyl-2-pyridin-2-yl-3,4-dihydro-2H-9-thia-1,4a-diaza-fluoren | 405.54. | 406,3 |
| 137 | 7-Ethoxy-4-phenylsulfanyl-2-pyridin-2-yl-3,4-dihydro-2H-9-thia-1,4a-diaza-fluoren | 419.56 | 420,3 |
| 138 | 7-Fluor-4-phenylsulfanyl-2-pyridin-2-yl-3,4-dihydro-2H-9-thia-1,4a-diaza-fluoren | 393.5 | 394,2 |
| 139 | 3-Naphthalin-2-yl-5-phenylsulfanyl-7-pyridin-2-yl-6,7-dihydro-5H-thiazolo[3,2-a]pyrimidin | 451.61 | 452,5 |
| 140 | 7-Phenyl-3-phenylazo-5-pyridin-2-yl-3,5,6,7-tetrahydro-pyrazolo[15-a]pyrimidin-2-ol | 396,47 | 397,4 |
| 141 | 7-Phenylsulfanyl-5-pyridin-2-yl-3,5,6,7-tetrahydro-pyrazolo[1,5-a]pyrimidine-3-carbansäureethylester | 380,5 | 381,4 |
| 142 | 3-Phenylazo-7-phenylsulfanyl-5-pyridin-2-yl-3,5,6,7-tetrahydro-pyrazolo[1,5-a]pyrinüdin-2-ol | 428,51 | 429,6 |
| 143 | 3-Brom-7-phenylsulfanyl-5-pyridin-2-yl-3,5,6,7-tetrahydro-pyrazolo[15-a]pyrimidin | 387,3 | 387,2 |
| 144 | 7-Phenylsuffanyl-5-pyridin-2-yl-3,5,6,7-tetrahydro-pyrazolo[1,5-a]pyrimidin-3-carbonitril. | 333,41 | 334,2 |
| 146 | 7-(3,4-Dimethoxy-phenyl)-2-methylsulfanyl-5-pyridin-2-yl-tetrahydro-pyrazolo[1,5-a]pyrimidin-3-carbonitril | 407,0 | 409,5/409,9 |
| 147 | 3-Brom-7-(3,4-dimethoxy-phenyl)-2-phenyl-5-pyridin-2-yl-tetrahydro-pyrazolo[1,5-a]pyrimidin | 490,9 | 492,6/494,4 |
| 148 | 3-Brom-7-(3,4-dimethoxy-phenyl)-2-phenyl-tetrahydro-pyrazolo[1,5-a]pyrimidin-5-carbonsäureethylester | 485,9 | 486,51488,4 |
| 149 | 3-Brom-7-(3,4-dimethoxy-phenyl)-5-(5-nitro-furan-2-yl)-2-phenyl-tetrahydro-pyrazolo[1,5-a]pyrimidin | 524,9 | 525,4/527,1 |
| 150 | 3-Cyano-7-(3,4-dimethoxy-phenyl)-2-methylsulfanyl-tetrahydro-pyrazolo[1,5-a]pyrimidin-5-carbonsäureethylester | 402,0 | 402,5 |
| 151 | 3-Cyano-7-(3,4-dimethoxy-phenyl)-tetrahydro-pyrazolo[1,5-a]pyrimidin-5-carbonsäureethylester | 356,0 | 357,2 |
| 153 | 7-(3,4-Dimethoxy-phenyl)-2-methylsulfanyl-5-(5-nitro-furan-2-yl)-tetrahydro-pyrazolo[1,5-a]pyrimidine-3-carbonitril | 441,5 | 442,1 |
| 154 | 7-(3,4-Dimethoxy-phenyl)-5-pyridin-2-yl-4,5,6,7-tetrahydro-pyrazolo[1,5-a]pyrimidine-3-carbonsäureethylester | 408,5 | 409,5 |

### Referenz-Beispiel 145

### 3-Brom-7-(3,4-dimethoxy-phenyl)-2-phenyl-5-pyridin-2-yl-4,5,6,7-tetrahydro-pyrazolo[1,5-a]pyrimidin-Dihydrochlorid

In einem 100 ml Einhalskolben wurden 1,5 g 5-Amino-4-brom-3-phenylpyrazol in Acetonitril vorgelegt, 1,55 g 3,4-Dimethoxystyrol, 0,88 g 2-Pyridylcarbaldehyd und 0,72 ml Trifluoressigsäure zugegeben und über Nacht bei Raumtemperatur gerührt. Die Reaktionslösung wurde komplett eingeengt. Das Rohprodukt wurde mit Reversed-Phase-HPLC aufgereinigt. HPLC-Säule: Macherey-Nagel, VP 100/21 Nucleosil 100-3 C18 HD (Ser.No. 0115186 Batch 23710123);
Gradient: Methanol (Riedel-deHaen, Chromasolv) / Wasser: Gradient (4 Stufen) von 60 bis 100 % Methanol in 37,5 min (Flow 10 ml/min, Injection volume: 1ml)
HPLC: Beckman SYSTEM GOLD, (Detector 166, Injector Endurance/SPARK, 125P Solvent Module, Fraktionssammler: Foxy200/ISCO)

Zur Hydrochloridbildung wurden 139 mg 3-Brom-7-(3,4-dimethoxy-phenyl)-2-phenyl-5-pyridin-2-yl-4,5,6,7-tetrahydro-pyrazolo[1,5-a]pyrimidin in 1,1 ml Methylethylketon gelöst und dann mit 6 µl H₂O und 7 µl TMSCI versetzt. Nach einiger Zeit fiel ein Feststoff aus. Nach Absaugen und waschen mit Ether erhielt man gelbe Kristalle, die im Vakuum getrocknet wurden.

¹H-NMR-Daten (600 MHz; DMSO-d6):
δ = 8.68 (m, 1 H), 8.27 (m, 1H), 7.92 (d, 1 H, J = 8.1 Hz), 7.74-7.67 (m, 3H), 7.39 (dd, 2H, J = 7.5, 7.5 Hz), 7.33 (t, 1 H, J = 7.2 Hz), 6.83 (d, 1H, J = 7.7 Hz), 6.78 (s, 1 H), 6.66 (d, 1 H, J = 9.0 Hz), 5.47 (m, 1 H), 5.03 (m, 1H), 3.71 (s, 3H), 3.69 (s, 3H), 2.67 (m, 1 H), 2.59 (m, 1H).

### Referenz-Beispiel 147

### 3-Brom-7-(3,4-dimethoxy-phenyl)-2-phenyl-5-pyridin-2-yl-tetrahydropyrazolo[1,5-a]pyrimidin, hergestellt nach dem oben angegebenen Verfahren

¹H-NMR-Daten (600 MHz; DMSO-d6):
δ = 8.34 (br. s, 1 H), 7.93 (d, 1 H, J = 3.8 Hz), 7.64 (s, 1 H), 7.14 (d, 1 H, J = 4.5 Hz), 6.81 (d, 1 H, J = 8.3 Hz), 6.71 (s, 1H), 6.60 (d, 1 H, J = 8.3 Hz), 5.40 (dd, 1 H, J = 4.5, 8.3 Hz), 5.09 (br. d, 1 H, J = 8.3 Hz), 3.70 (s, 3H), 3.68 (s, 3H), 2.59 (br. d, 1 H, J = 13.6 Hz), 2.50 (m, 1 H).

### Referenz-Beispiel 152

### 7-(3,4-Dimethoxy-phenyl)-5-pyridin-2-yl-4,5,6,7-tetrahydro-pyrazolo[1,5-a]pyrimidine-3-carbonitril-Dihydrochlorid

1 g (9,3 mmol) 3-Aminopyrazol-4-carbonitril wurden in 28 ml Acetonitril vorgelegt, 2,28 g (13,9 mmol) 3,4-Dimethoxystyrol, 1,29 g (12 mmol) Pyridin-2-carbaldehyd und 1,05 g (9,3 mmol) Trifluoeressigsäure zugegeben und über Nacht bei Raumtemperatur gerührt. Die dunkelbraune Lösung wurde komplett einrotiert und getrocknet. Das Produkt wurde über HPLC (Bedingungen wie oben angegeben) gereinigt.

Die so erhaltene Base (270 mg) wurde in Methanol suspendiert und dann mit 15µl H₂O und 208 µl TMSCI versetzt. Es entstand eine klare Lösung, die am Rotavapor eingeengt wurde. Die entstandenen gelbe Kristalle wurden im Vakuum getrocknet.

¹H-NMR-Daten (600 MHz; DMSO-d6):
δ=8.65 (d, 1 H, J = 4.8. Hz), 8.23 (t, 1 H, J = 7.2 Hz), 8.16 (m, 1 H), 7.86 (d, 1H), 7.70-7.65 (m, 2H), 6.77 (d, 1 H, J = 8.3 Hz), 6.68 (s, 1H), 6.53 (d, 1 H, J = 8.3 Hz), 5.43 (dd, 1 H, J = 4.5, 7.5 Hz), 5.08 (m, 1 H), 3.70 (s, 3H), 3.68 (s, 3H), 2.73 (m, 1 H), 2.63 (br. d, 1 H, J = 14.3 Hz).

### Referenz-Beispiel 56 (Dihydrochlorid)

In zu den Refernz-Beispielen 145 und 152 analoger Weise wurde das Dihydrochlorid von 7-(3,4-Dimethoxy-phenyl)-5-(5-nitro-thiophen-2-yl)-tetrahydro-pyrazolo[1,5-a]pyrimidin-3-carbonitril hergestellt.

¹H-NMR-Daten (600 MHz; DMSO-d6):
δ 8.65 (d, 1 H, J = 4.8 Hz), 8.23 (t, 1H, J = 7.2 Hz), 8.16 (m, 1 H), 7.86 (d, 1 H), 7.70-7.65 (m, 2H), 6.77 (d, 1 H, J = 8.3 Hz), 6.68 (s, 1 H), 6.53 (d, 1 H, J = 8.3 Hz), 5.43 (dd, 1 H, J = 4.5, 7.5 Hz), 5.08 (m, 1 H), 3.70 (s, 3H), 3.68 (s, 3H), 2.73 (m, 1H), 2.63 (br. d, 1 H, J = 14.3 Hz).

### Pharmakologische Untersuchungen:

Die Untersuchungen zur Bestimmung der NMDA-antagonistischen Wirkung der Verbindungen wurden an Hirnmembranhomogenaten (Homogenat von Rattenhirn ohne Cerebellum, Pons und Medulla oblongata von männlichen Wistar-Ratten (Charles River, Sulzfeld, Deutschland)) durchgeführt.

Hierzu wurden frisch präparierte Rattengehirne nach Abtrennen von Cerebellum, Pons und Medulla oblongata in 50 mmol/l Tris/HCl (pH 7,7) mit einem Polytron-Homogenisator (Modell PT3000, Kinematika AG, Littau, Schweiz) bei 6.000 Umdrehungen pro Minute (UPM) für 1 Minute unter Eiskühlung aufgeschlossen und anschließend für 15 Minuten bei 4 °C und 60.000 g zentrifugiert. Nach Dekantieren und Verwerfen des Überstandes, erneutem Aufnehmen in 50 mmol/l Tris/HCl (pH 7,7) und Aufschluß des Membranpellets mit einem Homogenisator bei 2.000 UPM für 1 Minute wurde erneut für 15 Minuten bei 4 °C und 60.000 g zentrifugiert. Der Überstand wurde wiederum verworfen und das Membranpellet in 50 mmol/l Tris/HCl (pH 7,7) homogenisiert (2.000 UPM für 1 Minute) und aliquotiert bei -70 °C eingefroren.

Für den Rezeptorbindungstest wurden jeweils Aliquote aufgetaut und anschließend für 15 Minuten bei 4 °C und 60.000 g zentrifugiert. Nach Dekantieren und Verwerfen des Überstandes wurde das Membranpellet für den Bindungstest mit Bindungstest-Puffer aufgenommen und homogenisiert (2.000 UPM für 1 Minute). Als Bindungstest-Puffer wurden 5 mmol/l Tris/HCI (pH 7,7) supplementiert mit 30 µmol/l Glycin und 100 µmol/l Glutaminsäure verwendet.

Als radioaktiv markierter Ligand wurde 1 nmol/l (³H)-(+)-MK801 ((5R,10S)-(+)-5-Methyl-10,11-dihydro-5H-dibenzo(a,d)cyclohepten-5,10-imin (NET-972, NEN, Köln, Deutschland)) zugegeben. Der Anteil an unspezifischer Bindung wurde in Anwesenheit von 10 µmol/l nicht radioaktiv markiertem (+)-MK801 (RBI/Sigma, Deisenhofen, Deutschland) bestimmt. In weiteren Ansätzen wurden die jeweiligen Verbindungen in Konzentrationsreihen zugegeben und die Verdrängung des radioaktiven Liganden aus seiner spezifischen Bindung am NMDA-Rezeptor ermittelt.

Die Ansätze wurden jeweils für 40 Minuten bei 25 °C inkubiert und anschließend zur Bestimmung des an das Hirnmembranhomogenat gebundenen radioaktiven Liganden mittels Filtration geerntet. Die durch die Filter zurückgehaltene Radioaktivität wurde nach Zugabe von Szintillator (Szintillator "Ready Protein", Beckmann Coulter GmbH, Krefeld, Deutschland) im β-Counter (Packard TRI-CARB Liquid Szintillation Analyzer 2000CA, Packard Instrument, Meriden, CT 06450, USA) vermessen.

Die resultierende prozentuale Hemmung der spezifischen Bindung des Liganden,(³H)-(+)-MK801 in Gegenwart von je 10 µmol/l der jeweiligen Verbindung dient als Maß für die Affinität dieser Verbindung zu der (+)-MK801-Bindungsstelle des ionotropen NMDA-Rezeptors. Die Affinität sind in Tabelle 2 als Mittelwerte von Doppelbestimmungen an ausgewählten Beispielen angegeben:

**Tabelle 2**

| **Referenz-Beispiel** | **% Hemmung** |
|---|---|
| 140 | 44 |
| 141 | 45 |
| 142 | 75 |
| 143 | 49 |
| 144 | 45 |

Für die Bestimmung der Hemmung des Nucleosid-Transport-Proteins wurden folgende Versuchsbedingungen gewählt:

100µl der Substanzlösung in wäßriger Lösung mit DMSO als Lösungsvermittler wurden mit 100µl [³H]NBI (N⁶-Benzyladenosin)1.5 nM, 1 00pl Puffer (50 mM Tris.HCl,pH 7.4) und 100µl wäßriger Suspension von Erythrocytenmembran 30 min bei 25°C inkubiert. Nach der Inkubation wurde das Testgemisch abfiltriert (Whatman GF/C Filter, mit 50 mM Tris.HCl nachgewaschen). Die Filter wurden in Röhrchen überführt, mit 3,5 ml Scintillations-Fluid versetzt und nach zwei Stunden im β-Counter gemessen. Die Meßergebnisse (Kᵢ-Wert bei 10 µM bzw. % Verdrängung bei 10 µM) sind in Tabelle 3 wiedergegeben.

**Tabelle 3**

| **Referenz-Beispiel Nr.** | **Ki-Wert [µM]** | **% Verdrängung bei 10 µM** |
|---|---|---|
| **39** | | 40 |
| **53** | 0,6 | |
| **54** | 1,3 | |
| **55** | 0,4 | |
| **56** | | 43 |
| **64** | 0,6 | |
| **111** | 0,3 | |
| **145** | 0,4 | |
| **153** | | 50 |
| **154** | | 41 |

Das Dihydrochlorid von Referenz-Beispiel 147 (hergestellt in zu den Referenz-Beispielen 145 und 152 analoger Weise aus Referenz-Beispiel 147) wurde auf seine Affinität gegenüber dem rekombinanten Human-Adensosin-A₃-Rezeptor in einem Verdrängungsassay (C. A. Salvatore et al., Proc. Natl. Acad. Sci. USA (1993), Vol. 90; 10365-10369) untersucht. Bei einer Ligandenkonzentration von 0.1 nM [¹²⁵I]AB-MECA (N⁶-(4-Amino-3-[¹²⁵I]iodbenzyladenosin) betrug die Verdrängung bei einer Konzentration von 3 µM 93 %.

### Pharmazeutische Formulierung eines Arzneimittels

1 g des Hydrochlorids von 3-Phenylazo-7-phenylsulfanyl-5-pyridin-2-yl-3,5,6,7-tetrahydro-pyrazolo[1,5-a]pyrimidin-2-ol wurde in 1 I Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur (II) 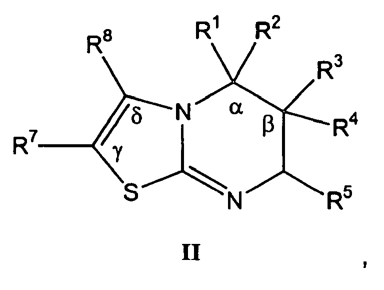 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl' oder -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 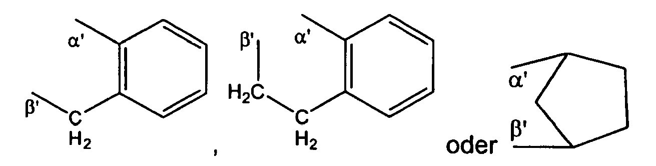 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NO₂, -SH, -OH, -CO₂H und CO₂Alkyl; Aryl' oder -(CH₂)ₖ-Aryl', wobei k = 1,2,3 oder 4 ist, Heterocyclyl oder C(=O)R¹¹ bedeutet;
R⁷ H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁸ H oder Aryl' bedeutet,
oder die Reste R⁷ und R⁸ zusammen Y bilden, wobei Y γ'-CR²¹=CR²²-CR²³=CR²⁴-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist;
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
R²¹, R²², R²³ und R²⁴ unabhängig voneinander H, Fluor, Chlor, Brom, Iod oder OR²⁸ bedeuten;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁸ H, Methyl oder Ethyl bedeutet;
Heterocyclyl Furan-2-yl, Furan-3-yl, Thien-2-yl, Thien-3-yl, Pyridin-2-y-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 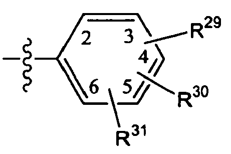 steht;
Aryl² für 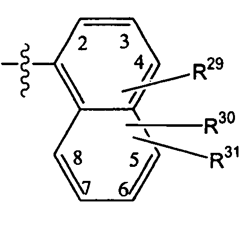 steht;
Aryl³ für 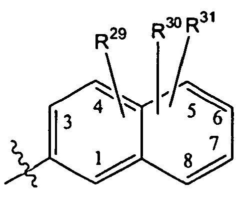 steht;
R²⁹, R³⁰ und R³¹ unabhängig voneinander H, C₁₋₆-[Alk], C₃₋₈-[Cycloalk], -(C₁₋₆-Alkyl)-C₃₋₈-[Cycloalk], [Ar], -(C₁₋₆-Alkyl)-[Ar], [Het], -(C₁₋₆-Alkyl)-[Het], F, Cl, Br, I, -CN,-NC, -OR³², -SR³³, -NO, -NO₂, NH₂, NHR³⁴, NR³⁵R³⁶,-N-OH, -N-OC₁₋₆-[Alk], -NHNH₂, -N=N-[Ar], -(C=O)R³⁷, 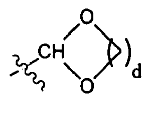 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalkl, -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)g- mit g = 4 oder 5 stehen;
R³⁷ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar] bedeutet; und
R³⁹ H, C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H, - CO₂-Alkyl,
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH, -CO₂H, - CO₂-Alkyl,
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht,
wobei die Verbindungen
3,4-Dihydro-2-(4-nitrophenyl)-4-phenyl-2H-pyrimido[2,1-b]benzothiazol,
3,4-Dihydro-4-(4-methylphenyl)-2-(4-nitrophenyl)-2H-pyrimido[2,1-b]benzothiazol
ausgenommen sind.

2. Verbindung nach Anspruch 1, **dadurch gekennzeichnet, daß**
R¹ und R² unabhängig voneinander H, O-R⁹, S-R¹⁰, unsubstituiertes oder einfach substituiertes oder mehrfach gleich oder verschieden substituiertes Methyl, Ethyl, n-Propyl, 2-Propyl, n-Butyl, tert.-Butyl oder n-Hexyl, wobei die Substituenten ausgewählt sind aus der Gruppe bestehend aus F, Cl, Br, I, -CN, -NC, -NH₂, -NO₂, -SH und -OH; Aryl' oder -CH₂-Aryl', wobei die Aryl'-Substituenten R²⁹, R³⁰ und R³¹ unabhängig voneinander H, Methyl, Ethyl, 2-Propyl, n-Butyl, tert.-Butyl, n-Hexyl, F, Cl, Br, I, OH, O-Methyl, O-Ethyl sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder Methyl, Ethyl, n-Propyl, 2-Propyl, n-Butyl, tert.-Butyl oder n-Hexyl bedeutet,
R³ und R⁴ H, Methyl oder Aryl¹ bedeutet, wobei die Aryl¹-Substituenten R²⁹, R³⁰ und R³¹ unabhängig voneinander H, Methyl oder O-Methyl sind, wobei mindestens einer der Reste R³ und R⁴ H ist,
oder
einer der Reste R¹ und R² zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 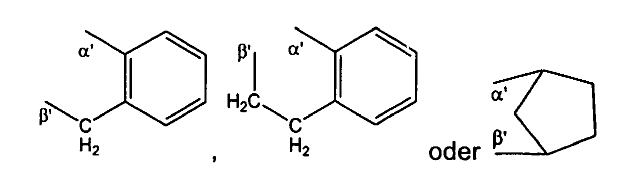 bedeutet, das mit α' **gekennzeichnet**e Ende von W mit dem mit α **gekennzeichnet**en Atom der Verbindung der allgemeinen Struktur (II) verbunden ist und das mit β' **gekennzeichnet**e Ende von W mit dem mit β **gekennzeichnet**en Atom der Verbindung der allgemeinen Struktur (II) verbunden ist, der andere Rest von R¹ und R² und der andere Rest von R³ und R⁴ jeweils H bedeuten;
R⁵ Methyl, Ethyl, n-Propyl, 2-Propyl, n-Butyl, iso-Butyl, sek.-Butyl, tert.-Butyl, -(CH₂)₄-OH, Cyclopropyl, wobei Cyclopropyl unsubstituiert oder einfach mit C(=O)OH, C(=O)O-Methyl oder C(=O)O-Ethyl substituiert ist, Cyclopentyl, Cyclohexyl, Aryl¹ oder -(CH₂)ₖ-Aryl¹, wobei die Aryl¹-Substituenten R²⁹, R³⁰ und R³¹ unabhängig voneinander H, -OH, -O-Methyl, O-C₆H₅, CH₃, CF₃ oder C(=O)OH sind und k = 1 oder 2 ist, Heterocyclyl oder C(=O)R¹¹ bedeutet;
R⁷ H, Aryl¹ mit R²⁹, R³⁰ und R³¹ gleich H, OH, S(O)_{q}R¹⁹, wobei q = 0 oder 2 ist, oder Methyl, Ethyl, n-Propyl, 2-Propyl, n-Butyl, iso-Butyl, sek.-Butyl oder tert.-Butyl bedeutet,
R⁸ H, Aryl¹, wobei die Aryl¹-Substituenten R²⁹, R³⁰ und R³¹ unabhängig voneinander H, Methyl oder Chlor sind, oder Aryl³ mit R²⁹, R³⁰ und R³¹ gleich H bedeutet,
oder
die Reste R⁷ und R⁸ zusammen Y bilden, wobei Y γ'-CR²¹=CR²²-CR²³=CR²⁴-δ' bedeutet und das mit γ' **gekennzeichnet**e Ende von Y mit dem mit γ **gekennzeichnet**en Atom der allgemeinen Struktur (II) verbunden ist und das mit δ' **gekennzeichnet**e Ende von Y mit dem mit δ **gekennzeichnet**en Atom der allgemeinen Struktur (II) verbunden ist;
R⁹ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclopentyl oder Cyclohexyl bedeutet oder -[(CH₂)ᵣ-O]ₛ-H mit r = 1, 2 oder 3 und s = 1 oder 2 bedeutet;
R¹⁰ Aryl¹ bedeutet;
R¹¹ Aryl¹ mit R²⁹, R³⁰ und R³¹ gleich H oder OR²⁵ bedeutet;
R¹⁹ Methyl oder Aryl¹ bedeutet, wobei einer der Aryl¹-Substiuenten R²⁹, R³⁰ und R³¹ gleich H oder -NO₂ ist und die beiden anderen Aryl¹-Substituenten von R²⁹, R³⁰ und R³¹ H sind,
R²¹ und R²³ H bedeuten;
R²² H, Fluor oder OR²⁸ bedeutet;
R²⁴ H oder Chlor bedeutet;
R²⁵ H, Methyl oder Ethyl bedeutet, wobei R²⁵ nicht H bedeutet, wenn zugleich R¹ Aryl und R² Alkyl bedeuten;
R²⁸ Methyl oder Ethyl bedeutet; und
Heterocyclyl Furan-2-yl, Furan-3-yl, Thien-2-yl, Thien-3-yl, Pyridin-2-y-, Pyridin-3-yl oder Pyridin-4-yl bedeutet, wobei Furanyl, Thienyl und Pyridinyl jeweils unsubstituiert oder einfach substituiert oder mehrfach gleich oder verschieden mit -NO₂, -CH₃ oder C(=O)OH substituiert sind.

3. Verbindung nach Anspruch 1 oder 2, **dadurch gekennzeichnet, daß**
R¹ und R² unabhängig voneinander H, O-CH₂-CH₂-OH, O-Cyclohexyl, S-Phenyl, Methyl, Phenyl, 3-Fluor-phenyl, 3-Brom-phenyl, 4-Brom-phenyl, 4-Chlor-phenyl, 4-Fluor-phenyl, 3-Methyl-phenyl, 4-Hydroxy-phenyl, 4-Methoxy-phenyl, 2,4-Dimethyl-phenyl, 3,4-Dimethoxyphenyl, 2,3,4-Trimethoxyphenyl, 2-Naphthyl oder -CH₂-Phenyl bedeuten,
R³ und R⁴ H, Methyl oder 4-Methoxy-phenyl bedeuten, wobei mindestens einer der Reste R³ und R⁴ H ist,
oder einer der Reste R¹ und R² zusammen mit einem der Reste R³ und R⁴
W bildet, wobei W α'-CH=CH-CH₂-β', α'-CH=CH-CH₂-CH₂-β', α'-O-(CH₂)ₘ-β' mit m = 2, 3, 4 oder 5, 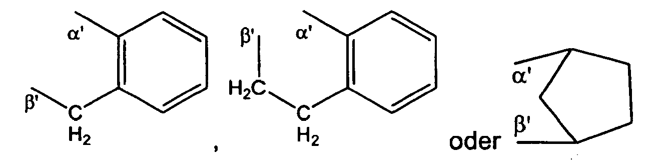 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² und der andere Rest von R³ und R⁴ jeweils H bedeuten;
R⁵ n-Propyl, n-Butyl, tert.-Butyl, -(CH₂)₄-OH, Cyclopropyl, Cycloprop-2-yl-1-carbonsäureethylester, Cyclohexyl, 4-Trifluorphenyl, 4-Phenoxy-phenyl, 2-Hydroxy-3-methoxy-phenyl, 4-Hydroxy-3-methoxy-phenyl, 3-Carboxy-2-hydroxy-phenyl, -(CH₂)₂-Phenyl, 5-Carboxyfuran-2-yl, 5-Methyl-furan-2-yl, 5-Nitro-furan-2-yl, 5-Nitro-thien-2-yl, Pyridin-2-yl, Pyridin-3-yl, C(=O)Phenyl, C(=O)OH oder C(=O)OEthyl bedeutet, wobei R⁵ nicht C(=O)OH bedeutet, wenn zugleich R¹ Aryl und R² Alkyl bedeuten;
R⁷ H, Phenyl, OH, -S-Methyl, -SO₂-(4-nitrophenyl) oder tert.-Butyl bedeutet,
R⁸ 4-Chlor-phenyl, 4-Methyl-phenyl oder 2-Naphthyl bedeutet,
oder
die Reste R⁷ und R⁸ zusammen Y bilden, wobei Y γ'-CR²¹=CH-CH=CH-δ' bedeutet und das mit γ' **gekennzeichnet**e Ende von Y mit dem mit γ **gekennzeichnet**en Atom der allgemeinen Struktur (II) verbunden ist und das mit δ' **gekennzeichnet**e Ende von Y mit dem mit δ **gekennzeichnet**en Atom der allgemeinen Struktur (II) verbunden ist; und
R²¹ Fluor, Methoxy oder Ethoxy bedeutet.

4. Verbindung nach irgendeinem der Ansprüche 1 bis 3, **dadurch gekennzeichnet, daß** die Verbindung ausgewählt ist aus:
• 2-(4-Nitro-phenylsulfonyl)-5-phenylsulfanyl-7-pyridin-2-yl-6,7-dihydro-5H-thiazolo[3,2-a]pyrimidin
• 3-(4-Chlor-phenyl)-5-phenylsulfanyl-7-pyridin-2-yl-6,7-dihydro-5H-thiazolo[3,2-a]pyrimidin
• 5-Phenylsulfanyl-7-pyridin-2-yl-3-p-tolyl-6,7-dihydro-5H-thiazolo[3,2-a]pyrimidin
• 7-Methoxy-4-phenylsulfanyl-2-pyridin-2-yl-3,4-dihydro-2H-9-thia-1,4a-diaza-fluoren
• 7-Ethoxy-4-phenylsulfanyl-2-pyridin-2-yl-3,4-dihydro-2H-9-thia-1,4a-diaza-fluoren
• 7-Fluor-4-phenylsulfanyl-2-pyridin-2-yl-3,4-dihydro-2H-9-thia-1,4a-diaza-fluoren
• 3-Naphthalin-2-yl-5-phenylsulfanyl-7-pyridin-2-yl-6,7-dihydro-5H-thiazolo[3,2-a]pyrimidin.

5. Verfahren zur Herstellung von Verbindungen der allgemeinen Struktur (II) oder pharmazeutisch annehmbarer Salze davon 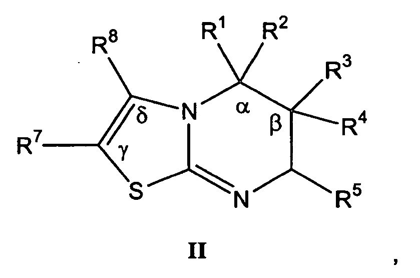 worin
R¹, R², R³, R⁴, R⁵, R⁷ und R⁸ wie in Anspruch 1 definiert sind,
**dadurch gekennzeichnet, daß**
ein Thiazolamin der allgemeinen Struktur (V1) 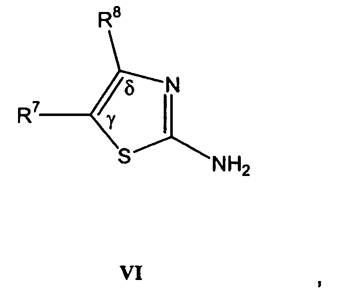 worin
R⁷und R⁸ wie oben in diesem Anspruch definiert sind mit der Maßgabe, daß, wenn R⁷ und R⁸ Y bilden, das mit γ' gekennzeichnete Ende von Y mit dem mit γ gekennzeichneten Atom des Thiazolamins der allgemeinen Struktur (VI) und das mit δ' gekennzeichnete Ende von Y mit dem mit δ gekennzeichneten Atom des Thiazolamins der allgemeinen Struktur (V1) verknüpft sind,
mit einem Aldehyd der allgemeinen Struktur (IV) 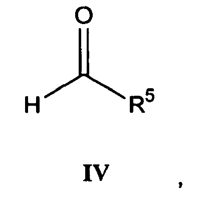 worin
R⁵ wie oben in diesem Anspruch definiert ist,
und einem Olefin der allgemeinen Struktur (V) 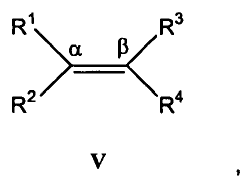 worin
R¹, R², R³ und R⁴ wie oben in diesem Anspruch definiert sind mit der Maßgabe, daß, wenn einer der Reste R¹ und R² zusammen mit einem der Reste R³ und R⁴ W bildet, das mit α' gekennzeichnete Ende von W mit dem α-Kohlenstoffatom des Olefins der allgemeinen Struktur (V) und das mit β' gekennzeichnete Ende von W mit dem β-Kohlenstoffatom des Olefins der allgemeinen Struktur (V) verbunden ist,
in Gegenwart einer Säure umgesetzt wird.

6. Verfahren nach Anspruch 5, **dadurch gekennzeichnet, daß** die Umsetzung des Heterocyclylamins der allgemeinen Struktur (VI) mit dem Aldehyd der allgemeinen Struktur (IV) und dem Olefin der allgemeinen Struktur (V) in einem Eintopf-Verfahren durchgeführt wird.

7. Verfahren nach Anspruch 5 oder 6, **dadurch gekennzeichnet, daß** die Säure Trifluoressigsäure ist.

8. Verfahren nach irgendeinem der Ansprüche 5 bis 7, **dadurch gekennzeichnet, daß** die Umsetzung in einem organischen Lösungsmittel bei einer Temperatur von 0 bis 100 °C und einer Reaktionszeit von 0,25 bis 12 h durchgeführt wird.

9. Verfahren nach einem der Ansprüche 5 bis 8, **dadurch gekennzeichnet, daß** die Reaktion bei einer Temperatur von 15 bis 40 °C durchgeführt wird.

10. Arzneimittel, enthaltend mindestens eine Verbindung der allgemeinen Struktur (II) 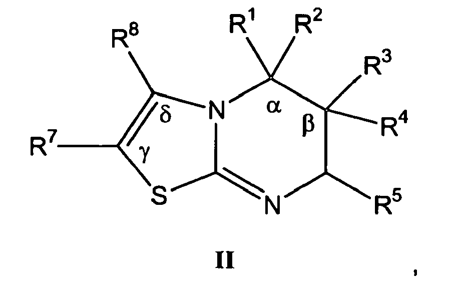 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl' oder -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴ W bildet, wobei W α'-CH=CH-CH₂-β', α'-CH=CH-CH₂-CH₂-β', α'-O-(CH₂)ₘ-β' mit m = 2, 3, 4 oder 5, 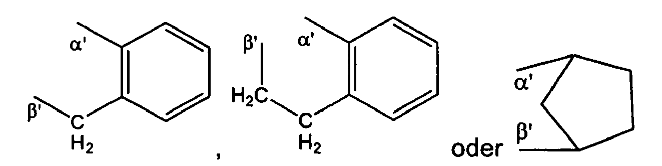 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NO₂, -SH, -OH, -CO₂H und CO₂Alkyl; Aryl' oder -(CH₂)ₖ-Aryl', wobei k = 1,2,3 oder 4 ist, Heterocyclyl oder C(=O)R¹¹ bedeutet;
R⁷ H, Aryl', OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁸ H oder Aryl' bedeutet, oder die Reste R⁷ und R⁸ zusammen Y bilden, wobei Y γ'-CR²¹=CR²²-CR²³=CR²⁴-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist;
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
R²¹, R²², R²³ und R²⁴ unabhängig voneinander H, Fluor, Chlor, Brom, Iod oder OR²⁸ bedeuten;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁸ H, Methyl oder Ethyl bedeutet;
Heterocyclyl Furan-2-yl, Furan-3-yl, Thien-2-yl, Thien-3-yl, Pyridin-2-y-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 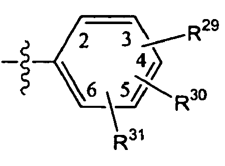 steht;
Aryl² für 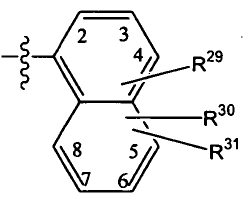 steht;
Aryl³ für 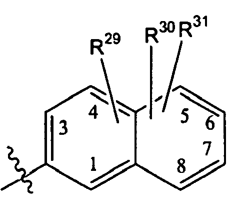 steht;
R²⁹, R³⁰ und R³¹ unabhängig voneinander H, C₁₋₆-[Alk], C₃₋₈-[Cycloalk], -(C₁₋₆-Alkyl)-C₃₋₈-[Cycloalk], [Ar], -(C₁₋₆-Alkyl)-[Ar], [Het], -(C₁₋₆-Alkyl)-[Het], F, Cl, Br, I, -CN,-NC, -OR³², -SR³³, -NO, -NO₂, NH₂, NHR³⁴, NR³⁵R³⁶,-N-OH, -N-OC₁₋₆-[Alk], -NHNH₂, -N=N-[Ar], -(C=O)R³⁷, 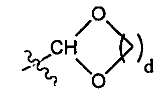 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)_{g}- mit g = 4 oder 5 stehen;
R³⁷ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar] bedeutet;
und
R³⁹ H, C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H, - CO₂-Alkyl,
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH, -CO₂H, - CO₂-Alkyl,
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrroldinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht.

11. Verwendung einer Verbindung der allgemeinen Struktur (II) 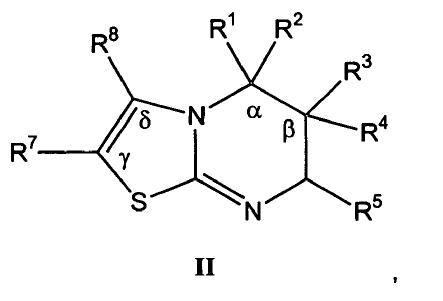 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl' oder -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴W bildet, wobei W α'-CH=CH-CH₂-β', α'-CH=CH-CH₂-CH₂-β', α'-O-(CH₂)ₘ-β' mit m = 2, 3, 4 oder 5, 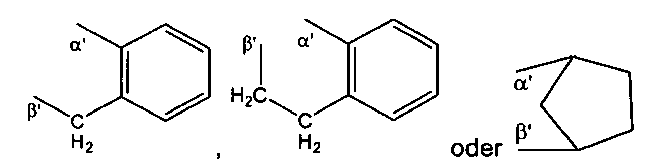 bedeutet, das mit α' gekennzeichnete Ende von W mit dem mit α gekennzeichneten Atom der Verbindung der allgemeinen Struktur (II) verbunden ist und das mit β' gekennzeichnete Ende von W mit dem mit β gekennzeichneten Atom der Verbindung der allgemeinen Struktur (II) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NO₂, -SH, -OH, -CO₂H und CO₂Alkyl; Aryl' oder -(CH₂)ₖ-Aryl', wobei k = 1,2,3 oder 4 ist, Heterocyclyl oder C(=O)R¹¹ bedeutet;
R⁷ H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁸ H oder Aryl' bedeutet,
oder die Reste R⁷ und R⁸ zusammen Y bilden, wobei Y γ'-CR²¹=CR²²-CR²³=CR²⁴-δ' bedeutet und das mit γ' gekennzeichnete Ende von Y mit dem mit γ gekennzeichneten Atom der allgemeinen Struktur (II) verbunden ist und das mit δ' gekennzeichnete Ende von Y mit dem mit δ gekennzeichneten Atom der allgemeinen Struktur (II) verbunden ist;
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
R²¹ R²², R²³ und R²⁴ unabhängig voneinander H, Fluor, Chlor, Brom, Iod oder OR²⁸ bedeuten;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁸ H, Methyl oder Ethyl bedeutet;
Heterocyclyl Furan-2-yl, Furan-3-yl, Thien-2-yl, Thien-3-yl, Pyridin-2-y-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 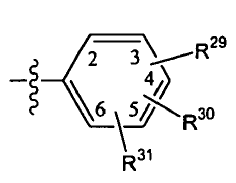 steht;
Aryl² für 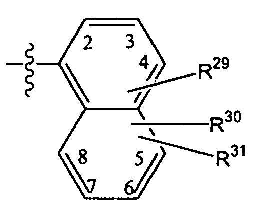 steht;
Aryl³ für 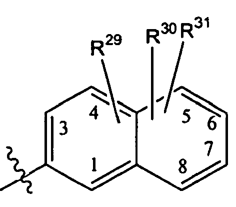 steht;
R²⁹, R³⁰ und R³¹ unabhängig voneinander H, C₁₋₆-[Alk], C₃₋₈-[Cycloalk], -(C₁₋₆-Alkyl)-C₃₋₈-[Cycloalk], [Ar], -(C₁₋₆-Alkyl)-[Ar], [Het], -(C₁₋₆-Alkyl)-[Het], F, Cl, Br, I, -CN,-NC, -OR³², -SR³³, -NO, -NO₂, NH₂, NHR³⁴ NR³⁵R³⁶,-N-OH, -N-OC₁₋₆-[Alk], -NHNH₂, -N=N-[Ar], -(C=O)R³⁷, 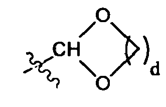 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)_{g}- mit g = 4 oder 5 stehen;
R³⁷ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar] bedeutet;
und
R³⁹ H, C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H,-CO₂-Alkyl,
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂. -SH, -OH, -CO₂H,-CO₂-Alkyl,
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrroldinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht,
zur Herstellung eines Arzneimittels zur Behandlung von Schmerz.

12. Verwendung einer Verbindung der allgemeinen Struktur (II), 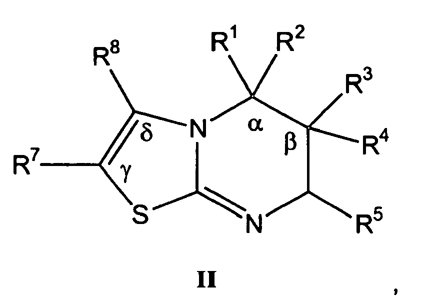 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin R¹, R², R³, R⁴, R⁵, R⁷ und R⁸ wie in Anspruch 11 definiert sind; zur Herstellung eines Arzneimittels zur Behandlung und/oder Prophylaxe von Epilepsie, Schizophrenie, neurodegenerativen Erkrankungen, insbesondere Morbus Alzheimer, Morbus Huntington und Morbus Parkinson, von cerebralen Ischämien und Infarkten, von Psychosen bedingt durch erhöhten Aminosäurespiegel, Hirnödemen, Unterversorgungszuständen des zentralen Nervensystems, insbesondere bei Hypoxien und Anoxien, von AIDS-Demens, von Encephalomyelitis, des Tourette-Syndroms, der perinatalen Asphyxie und/oder bei Tinnitus.

13. Pharmazeutische Zusammensetzung, die mindestens eine Verbindung der allgemeinen Struktur (II), 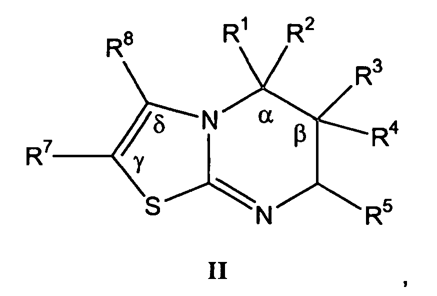 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin R¹, R², R³, R⁴, R⁵, R⁷und R⁸ wie in Anspruch 1 definiert sind; sowie mindestens einen pharmazeutischen Hilfsstoff enthält.

## Claims

1. A compound of the general structure (II) 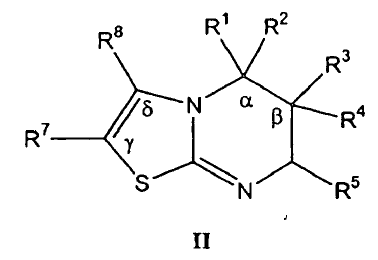 in the form as prepared or in the form of the acid(s) or base(s) thereof or in the form of one of the salts thereof, in particular the physiologically acceptable salts, or in the form of one of the solvates thereof, in particular the hydrates;
in the form of the racemate thereof, the pure stereoisomers, in particular enantiomers or diastereomers, or in the form of mixtures of the stereoisomers, in particular the enantiomers or diastereomers, in any desired mixing ratio;
in which
R¹ and R² mutually independently mean H, O-R⁹, S-R¹⁰, C₁₋₆-[alk], aryl' or -(C₁₋₆-alkyl)-aryl', wherein the aryl' substituents R²⁹, R³⁰ and R³¹ are mutually independently H, C₁₋₆-[alk], F, Cl, Br, I, OH, O-C₁₋₆-[alk], O-aryl¹ or O-CH₂-aryl¹, wherein one of the residues R¹ and R² is H and the other residue of R¹ and R² is not H or, in the event that one of the residues R¹ and R² means aryl', the other residue of R¹ and R² means H or C₁₋₆ alkyl,
R³ and R⁴ mean H, aryl' or -CH₂-aryl' or unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl, wherein at least one of the residues R³ and R⁴ is H, or one of the residues R¹ and R² together with one of the residues R³ and R⁴ forms W, wherein W means α'-CH=CH-CH₂₋β', α'-CH=CH-CH₂₋CH₂₋β', α'-O-(CH₂)ₘ-β' with m = 2, 3, 4 or 5, 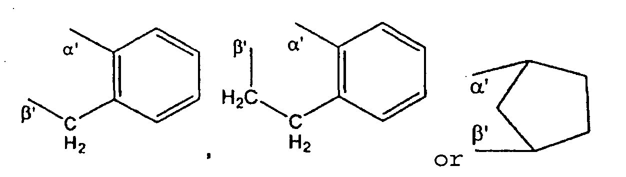 the end of W marked α' is joined to the atom marked α of the compound of the general structure (II) and the end of W marked β' is joined to the atom marked β of the compound of the general structure (II), the other residue of R¹ and R² means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, or sec.-hexyl and the other residue of R³ and R⁴ means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl;
R⁵ means methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl, which are in each case unsubstituted or monosubstituted or identically or differently polysubstituted with a substituent selected from the group consisting of F, Cl, Br, I, -CN, -NC, -NO₂, -SH, -OH, -CO₂H and CO₂-alkyl; aryl' or-(CH₂)ₖ-aryl', wherein k = 1, 2, 3 or 4, heterocyclyl or C(=O)R¹¹;
R⁷ means H, aryl¹, OR¹⁸ S(O)_{q}R¹⁹, wherein q = 0, 1 or 2, or unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl,
R⁸ means H or aryl',
or the residues R⁷ and R⁸ together form Y, wherein Y means γ'-CR²¹=CR²²-CR²³=CR²⁴-δ' and the end of Y marked γ' is joined to the atom marked γ of the general structure (II) and the end of Y marked δ' is joined to the atom marked δ of the general structure (II);
R⁹ means unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl or -[CH₂)ᵣ-O]ₛ-H with r = 1, 2, 3, 4, 5 or 6 and s = 1, 2, 3, 4, 5 or 6;
R¹⁰ means aryl';
R¹¹ means aryl' or OR²⁵;
R¹⁸ means H or methyl;
R¹⁹ means H, aryl¹ or in each case unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl;
R²¹, R²², R²³ and R²⁴ mutually independently mean H, fluorine, chlorine, bromine, iodine or OR²⁸;
R²⁵ means H, methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl, wherein R²⁵ does not mean H if, at the same time, R¹ means aryl and R² means alkyl;
R²⁸ means H, methyl or ethyl;
heterocyclyl means furan-2-yl, furan-3-yl, thien-2-yl, thien-3-yl, pyridin-2-yl, pyridin-3-yl or pyridin-4-yl, wherein furanyl, thienyl and pyridinyl are in each case unsubstituted or monosubstituted or identically or differently polysubstituted with a substituent selected from the group consisting of F, Cl, Br, I, -CN, -NO₂, -CO₂H and -CO₂-alkyl;
aryl' means aryl¹, aryl² or aryl³;
aryl¹ denotes 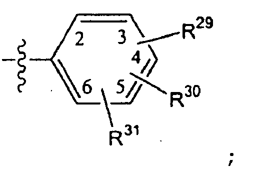
aryl² denotes 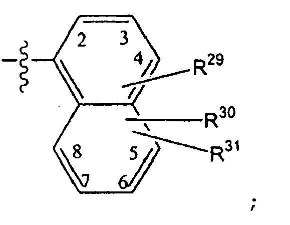
aryl³ denotes 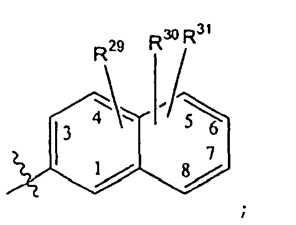
R²⁹, R³⁰ and R³¹ mutually independently mean H, C₁₋₆-[alk], (C₃₋₈-[cycloalk], - (C₁₋₆-alkyl)-C₃₋₈-[cycloalk] , [Ar], - (C₁₋₆-alkyl)-[Ar], [het], -(C₁₋₆-alkyl)-[het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N- [Ar], -(C=O)R³⁷, 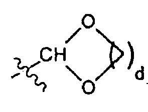 with d = 1, 2 or 3, or -(C=S)R³⁷ and may be in any desired ring position;
R³² and R³³ mutually independently mean H, -C₁₋₆-[alk], -C₃₋₈-[cycloalk], - (C₁₋₆-alkyl) -C₃₋₈-[cycloalk], -[Ar], -(C₁₋₆-alkyl)-[Ar], -[het], -(C₁₋₆-alkyl)-[het], (C=O)R³⁸, -[(CH₂)_{w}O]_{z}-H or -[(CH₂)_{w}-O]_{z}-C₁₋₆-[alk] with w = 1, 2, 3 or 4 and z = 1, 2, 3, 4 or 5;
R³⁴ means C₁₋₆-[alk], -CH₂-[Ar] or -(C=O)O-tert.-butyl;
R³⁵ and R³⁶ mutually independently mean C₁₋₆- [alk] or together denote (CH₂)_{g}- with g = 4 or 5;
R³⁷ means H, -C₁₋₆-[alk], -C₃₋₈-[cycloalk], - (C₁₋₆-alkyl) -C₃₋₈- [cycloalk], - [Ar], - (C₁₋₆-alkyl) - [Ar], - [het], -(C₁₋₆-alkyl)-[het], -OR³⁹, -NH₂, -NHR³⁴, -NR³⁵R³⁶;
R³⁸ H, -C₁₋₆-[alk], -C₃₋₈- [cycloalk], - (C₁₋₆-alkyl) -C₃₋₈- [cycloalk], - [Ar], - (C₁₋₆-alkyl) - [Ar] ;
and
R³⁹ means H, -C₁₋₆-[alk], -C₃₋₈-[cycloalk], - (C₁₋₆-alkyl)-C₃₋₈-[cycloalk], -[Ar], - (C₁₋₆-alkyl)-[Ar], -[het] or - (C₁₋₆-alkyl)-[het],
[alk] denotes an acyclic, saturated or unsaturated, branched or linear hydrocarbon residue which may be unsubstituted or monosubstituted or identically or differently polysubstituted,
wherein the substituents may be selected from the group consisting of F, Cl, Br, I, -CN, -NC, -NH₂, -NO₂, -SH, -OH, -CO₂H, -CO₂-alkyl,
[cycloalk] denotes a cyclic, saturated or unsaturated hydrocarbon residue which may be unsubstituted or monosubstituted or identically or differently polysubstituted and optionally benzo-fused,
wherein the substituents may be selected from the group consisting of F, Cl, Br, I, -CN, -NC, -NH₂, -NO₂, -SH, -OH, -CO₂H, -CO₂-alkyl,
[Ar] denotes an unsubstituted residue selected from the group consisting of phenyl, naphthyl, anthracenyl and biphenyl;
[het] denotes an unsubstituted heterocyclyl residue selected from the group consisting of pyrrolidinyl, tetrahydrofuryl, piperidinyl, piperazinyl and morpholinyl or denotes an unsubstituted heteroaryl residue selected from the group consisting of pyrrolyl, pyrazolyl, imidazolyl, pyridazinyl, pyrimidinyl, pyrazinyl, furanyl, thienyl and pyridinyl,
wherein the compounds
3,4-dihydro-2-(4-nitrophenyl)-4-phenyl-2H-pyrimido[2,1-b]benzothiazole,
3,4-dihydro-4-(4-methylphenyl)-2-(4-nitrophenyl)-2H-pyrimido[2,1-b]benzothiazole
are excepted.

2. A compound according to claim 1, **characterised in that**
R¹ and R² mutually independently mean H, O-R⁹, S-R¹⁰, unsubstituted or monosubstituted or identically or differently polysubstituted substituted methyl, ethyl, n-propyl, 2-propyl, n-butyl, tert.-butyl or n-hexyl, wherein the substituents are selected from the group consisting of F, Cl, Br, I, -CN, -NC, -NH₂, -NO₂, -SH and -OH; aryl' or -CH₂-aryl', wherein the aryl' substituents R²⁹, R³⁰ and R³¹ are mutually independently H, methyl, ethyl, 2-propyl, n-butyl, tert.-butyl, n-hexyl, F, Cl, Br, I, OH, O-methyl, O-ethyl,
wherein one of the residues R¹ and R² is H and the other residue of R¹ and R² is not H or, in the event that one of the residues R¹ and R² means aryl', the other residue of R¹ and R² means H or methyl, ethyl, n-propyl, 2-propyl, n-butyl, tert.-butyl or n-hexyl,
R³ and R⁴ mean H, methyl or aryl¹, wherein the aryl¹ substituents R²⁹, R³⁰ and R³¹ are mutually independently H, methyl or O-methyl, wherein at least one of the residues R³ and R⁴ is H,
or
one of the residues R¹ and R² together with one of the residues R³ and R⁴ forms W, wherein W means α'-CH=CH-CH₂-β', α'-CH=CH-CH₂-CH₂-β', α'-O-(CH₂)ₘ-β' with m = 2, 3, 4 or 5, 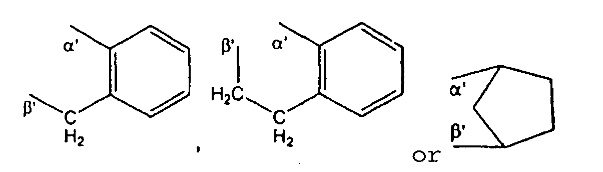 the end of W marked α' is joined to the atom marked α of the compound of the general structure (II) and the end of W marked β' is joined to the atom marked β of the compound of the general structure (II), the other residue of R¹ and R² and the other residue of R³ and R⁴ in each case mean H;
R⁵ means methyl, ethyl, n-propyl, 2-propyl, n-butyl, iso-butyl, sec.-butyl, tert.-butyl, -(CH₂)₄-OH, cyclopropyl, wherein cyclopropyl is unsubstituted or monosubstituted with C(=O)OH, C(=O)O-methyl or C(=O)O-ethyl, cyclopentyl, cyclohexyl, aryl¹ or -(CH₂)ₖ-aryl¹, wherein the aryl¹ substituents R²⁹, R³⁰ and R³¹ are mutually independently H, -OH, -O-methyl, O-C₆H₅, CH₃, CF₃ or C(=O)OH and k = 1 or 2, heterocyclyl or C(=O)R¹¹;
R⁷ means H, aryl¹ with R²⁹, R³⁰ and R³¹ being H, OH, S(O)_{q}R¹⁹, wherein q = 0 or 2, or methyl, ethyl, n-propyl, 2-propyl, n-butyl, iso-butyl, sec.-butyl or tert.-butyl,
R⁸ means H, aryl¹, wherein the aryl¹ substituents R²⁹, R³⁰ and R³³ are mutually independently H, methyl or chlorine, or aryl³ with R²⁹, R³⁰ and R³¹ being H, or
the residues R⁷ and R⁸ together form Y, wherein Y means γ'-CR²¹=CR²²-CR²³=CR²⁴-δ' and the end of Y marked γ' is joined to the atom marked γ of the general structure (II) and the end of Y marked δ' is joined to the atom marked δ of the general structure (II);
R⁹ means methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclopentyl or cyclohexyl means or -[CH₂)ᵣ-O]ₛ-H with r = 1, 2 or 3 and s = 1 or 2;
R¹⁰ means aryl¹;
R¹¹ means aryl¹ with R²⁹, R³⁰ and R³¹ being H or OR²⁵;
R¹⁹ means methyl or aryl¹, wherein one of the aryl¹ substituents R²⁹, R³⁰ and R³¹ is H or -NO₂ and the other two aryl¹ substituents of R²⁹, R³⁰ and R³¹ are H,
R²¹ and R²³ mean H;
R²² means H, fluorine or OR²⁸;
_{R}²⁴ means H or chlorine;
_{R}²⁵ means H, methyl or ethyl, wherein R²⁵ does not mean H if, at the same time, R¹ means aryl and R² means alkyl;
R²⁸ means methyl or ethyl; and
heterocyclyl means furan-2-yl, furan-3-yl, thien-2-yl, thien-3-yl, pyridin-2-yl, pyridin-3-yl or pyridin-4-yl, wherein furanyl, thienyl and pyridinyl are in each case unsubstituted or monosubstituted or identically or differently polysubstituted with -NO₂, -CH₃ or C(=O)OH.

3. A compound according to claim 1 or claim 2,
**characterised in that**
R¹ and R² mutually independently mean H, O-CH₂-CH₂-OH, O-cyclohexyl, S-phenyl, methyl, phenyl, 3-fluorophenyl, 3-bromophenyl, 4-bromophenyl, 4-chlorophenyl, 4-fluorophenyl, 3-methylphenyl, 4-hydroxyphenyl, 4-methoxyphenyl, 2,4-dimethylphenyl, 3,4-dimethoxyphenyl, 2,3,4-trimethoxyphenyl, 2-naphthyl or -CH₂-phenyl,
R³ and R⁴ mean H, methyl or 4-methoxyphenyl, wherein at least one of the residues R³ and R⁴ is H, or one of the residues R¹ and R² together with one of the residues R³ and R⁴ forms W, wherein W means α'-CH=CH-CH₂-β', α'-CH=CH-CH₂-CH₂-β', α'-O-(CH₂)ₘ-β' with m = 2, 3, 4 or 5, 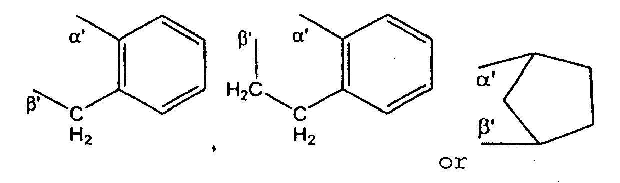 the end of W marked α' is joined to the atom marked α of the compound of the general structure (II) and the end of W marked β' is joined to the atom marked β of the compound of the general structure (II), the other residue of R¹ and R² and the other residue of R³ and R⁴ in each case mean H;
R⁵ means n-propyl, n-butyl, tert.-butyl, -(CH₂)₄-OH, cyclopropyl, cycloprop-2-yl 1-carboxylic acid ethyl ester, cyclohexyl, 4-trifluorophenyl, 4-phenoxyphenyl, 2-hydroxy-3-methoxyphenyl, 4-hydroxy-3-methoxyphenyl, 3-carboxy-2-hydroxyphenyl, -(CH₂)₂-phenyl, 5-carboxyfuran-2-yl, 5-methylfuran-2-yl, 5-nitrofuran-2-yl, 5-nitrothien-2-yl, pyridin-2-yl, pyridin-3-yl, C(=O)phenyl, C(=O)OH or C(=O)O-ethyl, wherein R⁵ does not mean C(=O)OH if, at the same time, R¹ means aryl and R² means alkyl;
R⁷ means H, phenyl, OH, -S-methyl, -SO₂-(4-nitrophenyl) or tert.-butyl,
R⁸ means 4-chlorophenyl, 4-methylphenyl or 2-naphthyl,
or
the residues R⁷ and R⁸ together form Y, wherein Y means Y'-CR²¹=CH-CH=CH-δ' and the end of Y marked Y' is joined to the atom marked Y of the general structure (II) and the end of Y marked δ' is joined to the atom marked δ of the general structure (II); and
R²¹ means fluorine, methoxy or ethoxy.

4. A compound according to any one of claims 1 to 3, **characterised in that** the compound is selected from among:
• 2-(4-nitrophenylsulfonyl)-5-phenylsulfanyl-7-pyridin-2-yl-6,7-dihydro-5H-thiazolo[3,2-a]pyrimidine
• 3-(4-chlorophenyl)-5-phenylsulfanyl-7-pyridin-2-yl-6,7-dihydro-5H-thiazolo[3,2-a]pyrimidine
• 5-phenylsulfanyl-7-pyridin-2-yl-3-p-tolyl-6,7-dihydro-5H-thiazolo[3,2-a]pyrimidine
• 7-methoxy-4-phenylsulfanyl-2-pyridin-2-yl-3,4-dihydro-2H-9-thia-1,4a-diaza-fluorene
• 7-ethoxy-4-phenylsulfanyl-2-pyridin-2-yl-3,4-dihydro-2H-9-thia-1,4a-diaza-fluorene
• 7-fluoro-4-phenylsulfanyl-2-pyridin-2-yl-3,4-dihydro-2H-9-thia-1,4a-diaza-fluorene
• 3-naphthalen-2-yl-5-phenylsulfanyl-7-pyridin-2-yl-6,7-dihydro-5H-thiazolo[3,2-a]pyrimidine.

5. A method for producing compounds of the general structure (II) or pharmaceutically acceptable salts thereof 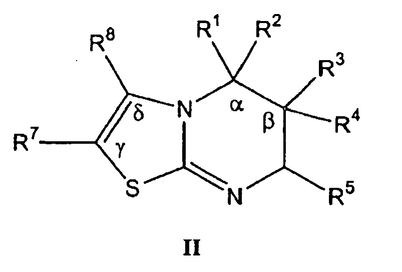 in which
R¹, R², R³, R⁴, R⁵, R⁷ and R⁸ are defined as in claim 1,
**characterised in that**
a thiazolamine of the general structure (VI) 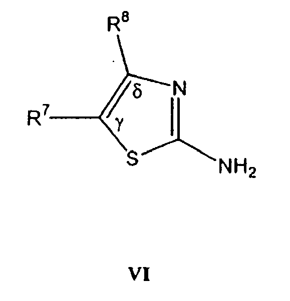 in which
R⁷ and R⁸ are defined as above in the present claim providing that, if R⁷ and R⁸ form Y, the end of Y marked γ' is linked with the atom marked γ of the thiazolamine of the general structure (VI) and the end of Y marked δ' is linked with the atom marked δ of the thiazolamine of the general structure (VI),
is reacted in the presence of an acid with an aldehyde of the general structure (IV) 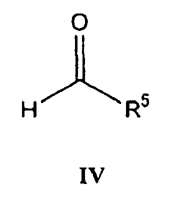 in which
R⁵ is defined as above in the present claim,
and an olefin of the general structure (V) 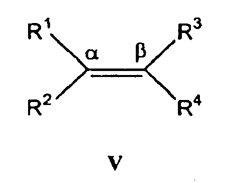 in which
R¹, R², R³ and R⁴ are defined as above in the present claim providing that, if one of the residues R¹ and R² together with one of the residues R³ and R⁴ forms W, the end of W marked α' is joined to the α carbon atom of the olefin of the general structure (V) and the end of W marked β' is joined to the β carbon atom of the olefin of the general structure (V).

6. A method according to claim 5, **characterised in that** the reaction of the heterocyclylamine of the general structure (VI) with the aldehyde of the general structure (IV) and the olefin of the general structure (V) is performed in a single-vessel reaction.

7. A method according to claim 5 or claim 6, **characterised in that** the acid is trifluoroacetic acid.

8. A method according to any one of claims 5 to 7, **characterised in that** the reaction is performed in an organic solvent at a temperature of 0 to 100°C and a reaction time of 0.25 to 12 h.

9. A method according to any one of claims 5 to 8, **characterised in that** the reaction is performed at a temperature of 15 to 40°C.

10. A medicament containing at least one compound of the general structure (II) 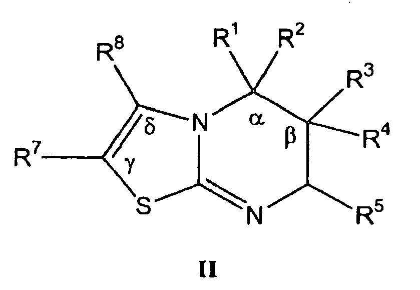 in the form as prepared or in the form of the acid(s) or base(s) thereof or in the form of one of the salts thereof, in particular the physiologically acceptable salts, or in the form of one of the solvates thereof, in particular the hydrates;
in the form of the racemate thereof, the pure stereoisomers, in particular enantiomers or diastereomers, or in the form of mixtures of the stereoisomers, in particular the enantiomers or diastereomers, in any desired mixing ratio;
in which
R¹ and R² mutually independently mean H, O-R⁹, S-R¹⁰, C₁₋₆-[alk], aryl' or -(C₁₋₆-alkyl) -aryl', wherein the aryl' substituents R²⁹, R³⁰ and R³¹ are mutually independently H, C₁₋₆-[alk], F, Cl, Br, I, OH, O-C₁₋₆-[alk], O-aryl¹ or O-CH₂-aryl¹, wherein one of the residues R¹ and R² is H and the other residue of R¹ and R² is not H or, in the event that one of the residues R¹ and R² means aryl', the other residue of R¹ and R² means H or C₁₋₆ alkyl,
R³ and R⁴ mean H, aryl' or -CH₂-aryl' or unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl, wherein at least one of the residues R³ and R⁴ is H, or one of the residues R¹ and R² together with one of the residues R³ and R⁴ forms W, wherein W means α'-CH=CH-CH₂-β', α'-CH=CH-CH₂-CH₂-β', α'-O-(CH₂)ₘ-β' with m = 2, 3, 4 or 5, 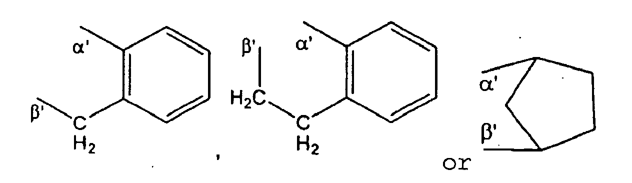 the end of W marked α' is joined to the atom marked α of the compound of the general structure (II) and the end of W marked β' is joined to the atom marked β of the compound of the general structure (II), the other residue of R¹ and R² means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, or sec.-hexyl and the other residue of R³ and R⁴ means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl;
R⁵ means methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl, which are in each case unsubstituted or monosubstituted or identically or differently polysubstituted with a substituent selected from the group consisting of F, Cl, Br, I, -CN, -NC, -NO₂, -SH, -OH, -CO₂H and CO₂-alkyl; aryl' or- (CH₂)ₖ-aryl', wherein k = 1, 2, 3 or 4, heterocyclyl or C(=O)R¹¹;
R⁷ means H, aryl¹, OR¹⁸, S(O)_{q}R¹⁹, wherein q = 0, 1 or 2, or unsubstituted methyl, ethyl, n-propyl, 2-propyl, n-butyl, iso-butyl, sec.-butyl, tert.-butyl, n-amyl, iso-amyl, sec.-amyl, n-'hexyl, iso-hexyl or sec.-hexyl,
R⁸ means H or aryl', or the residues R⁷ and R⁸ together form Y, wherein Y means γ'-CR²¹=CR²²-CR²³=CR²⁴-δ' and the end of Y marked γ' is joined to the atom marked γ of the general structure (II) and the end of Y marked δ' is joined to the atom marked δ of the general structure (II);
R⁹ means unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl or - [CH₂)ᵣ-O]ₛ-H with r = 1, 2, 3, 4, 5 or 6 and s = 1, 2, 3, 4, 5 or 6;
R¹⁰ means aryl';
R¹¹ means aryl' or OR²⁵;
R¹⁸ means H or methyl;
R¹⁹ means H, aryl¹ or in each case unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl;
R²¹, R²², R²³ and R²⁴ mutually independently mean H, fluorine, chlorine, bromine, iodine or OR²⁸;
R²⁵ means H, methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl, wherein R²⁵ does not mean H if, at the same time, R¹ means aryl and R² means alkyl;
R²⁸ means H, methyl or ethyl;
heterocyclyl means furan-2-yl, furan-3-yl, thien-2-yl, thien-3-yl, pyridin-2-yl, pyridin-3-yl or pyridin-4-yl, wherein furanyl, thienyl and pyridinyl are in each case unsubstituted or monosubstituted or identically or differently polysubstituted with a substituent selected from the group consisting of F, Cl, Br, I, -CN, -NO₂, -CO₂H and -CO₂-alkyl;
aryl' means aryl¹, aryl² or aryl³;
aryl¹ denotes 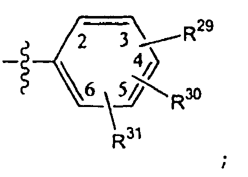
aryl² denotes 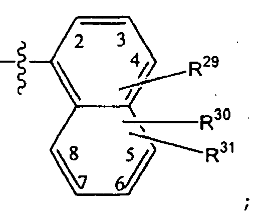
aryl³ denotes 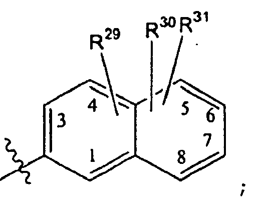
R²⁹, R³⁰ and R³¹ mutually independently mean H, C₁₋₆-[alk], (C₃₋₈-[cycloalk], - (C₁₋₆-alkyl) -C₃₋₈-[cycloalk], [Ar], - (C₁₋₆-alkyl)-[Ar], [het], -(C₁₋₆-alkyl)-[het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N- [Ar], -(C=O)R³⁷, 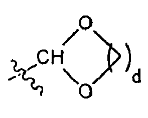 with d = 1, 2 or 3, or -(C=S)R³⁷ and may be in any desired ring position;
R³² and R³³ mutually independently mean H, -C₁₋₆-[alk], -C₃₋₈-[cycloalk], - (C₁₋₆-alkyl)-C₃₋₈-[cycloalk], -[Ar], - (C₁₋₆-alkyl)-[Ar], -[het], -(C₁₋₆-alkyl)-[het], (C=O)R³⁸, -[(CH₂)_{w}O]_{z}-H or -[(CH₂)_{w}-O]_{z}-C₁₋₆-[alk] with w = 1, 2, 3 or 4 and z = 1, 2, 3, 4 or 5;
R³⁴ means C₁₋₆-[alk], -CH₂- [Ar] or -(C=O)O-tert.-butyl;
R³⁵ and R³⁶ mutually independently mean C₁₋₆-[alk] or together denote -(CH₂)_{g}- with g = 4 or 5;
R³⁷ means H, -C₁₋₆-[alk], -C₃₋₈- [cycloalk], - (C₁₋₆-alkyl)-C₃₋₈-[cycloalk], -[Ar], -(C₁₋₆-alkyl)-[Ar], [het], - (C₁₋₆-alkyl)-[het], -OR³⁹, -NH₂, -NHR³⁴, -NR³⁵R³⁶ ;
R³⁸ H, -C₁₋₆-[alk], -C₃₋₈-[cycloalk], - (C₁₋₆-alkyl) -C₃₋₈-[cycloalk], -[Ar], - (C₁₋₆-alkyl)-[Ar];
and
R³⁹ means H, -C₁₋₆-[alk], -C₃₋₈-[cycloalk], - (C₁₋₆-alkyl) -C₃₋₈-[cycloalk], -[Ar], -(C₁₋₆-alkyl)-[Ar], -[het] or - (₁₋₆-alkyl)-[het],
[alk] denotes an acyclic, saturated or unsaturated, branched or linear hydrocarbon residue which may be unsubstituted or monosubstituted or identically or differently polysubstituted,
wherein the substituents may be selected from the group consisting of F, Cl, Br, I, -CN, -NC, -NH₂, -NO₂, -SH, -OH, -CO₂H, -CO₂-alkyl,
[cycloalk] denotes a cyclic, saturated or unsaturated hydrocarbon residue which may be unsubstituted or monosubstituted or identically or differently polysubstituted and optionally benzo-fused,
wherein the substituents may be selected from the group consisting of F, Cl, Br, I, -CN, -NC, -NH₂, -NO₂, -SH, -OH, -CO₂H, -CO₂-alkyl,
[Ar] denotes an unsubstituted residue selected from the group consisting of phenyl, naphthyl, anthracenyl and biphenyl;
[het] denotes an unsubstituted heterocyclyl residue selected from the group consisting of pyrrolidinyl, tetrahydrofuryl, piperidinyl, piperazinyl and morpholinyl or denotes an unsubstituted heteroaryl residue selected from the group consisting of pyrrolyl, pyrazolyl, imidazolyl, pyridazinyl, pyrimidinyl, pyrazinyl, furanyl, thienyl and pyridinyl.

11. Use of a compound of the general structure (II) 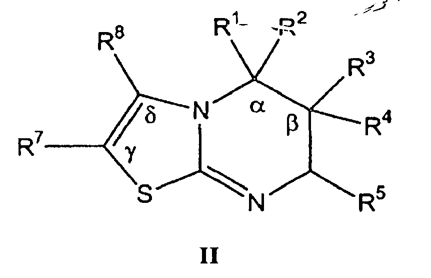 in the form as prepared or in the form of the acid(s) or base(s) thereof or in the form of one of the salts thereof, in particular the physiologically acceptable salts, or in the form of one of the solvates thereof, in particular the hydrates;
in the form of the racemate thereof, the pure stereoisomers, in particular enantiomers or diastereomers, or in the form of mixtures of the stereoisomers, in particular the enantiomers or diastereomers, in any desired mixing ratio; in which
R¹ and R² mutually independently mean H, O-R⁹, S-R¹⁰, C₁₋₆-[alk], aryl' or -(C₁₋₆-alkyl)-aryl', wherein the aryl' substituents R²⁹, R³⁰ and R³¹ are mutually independently H, C₁₋₆-[alk], F, Cl, Br, I, OH, O-C₁₋₆-[alk], O-aryl¹ or O-CH₂-aryl¹, wherein one of the residues R¹ and R² is H and the other residue of R¹ and R² is not H or, in the event that one of the residues R¹ and R² means aryl', the other residue of R¹ and R² means H or C₁₋₆ alkyl,
R³ and R⁴ mean H, aryl' or -CH₂-aryl' or unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl,
wherein at least one of the residues R³ and R⁴ is H, or one of the residues R¹ and R² together with one of the residues R³ and R⁴ forms W, wherein W means α'-CH=CH-CH₂-β', α'-CH=CH-CH₂-CH₂-β', α'-O-(CH₂)ₘ-β' with m = 2, 3, 4 or 5, 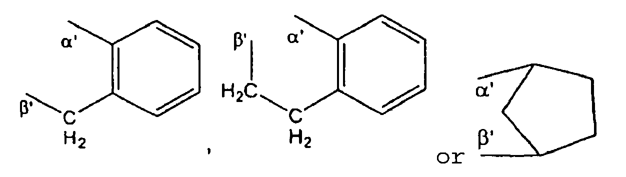 the end of W marked α' is joined to the atom marked α of the compound of the general structure (II) and the end of W marked β' is joined to the atom marked β of the compound of the general structure (II), the other residue of R¹ and R² means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, or sec.-hexyl and the other residue of R³ and R⁴ means H or methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl;
R⁵ means methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl, which are in each case unsubstituted or monosubstituted or identically or differently polysubstituted with a substituent selected from the group consisting of F, Cl, Br, I, -CN, -NC, -NO₂, -SH, -OH, -CO₂H and CO₂-alkyl; aryl' or-(CH₂)ₖ-aryl', wherein k = 1, 2, 3 or 4, heterocyclyl or C(=O)R¹¹;
R⁷ means H, aryl¹ OR¹⁸, S(O)_{q}R¹⁹, wherein q = 0, 1 or 2, or unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl,
R⁸ means H or aryl',
or the residues R⁷ and R⁸ together form Y, wherein Y means γ'-CR²¹=CR²²-CR²³=CR²⁴-δ' and the end of Y marked γ' is joined to the atom marked γ of the general structure (II) and the end of Y marked δ' is joined to the atom marked δ of the general structure (II);
R⁹ means unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl, sec.-hexyl, cyclopropyl, cyclobutyl, cyclopentyl, cyclohexyl or cycloheptyl or - [CH₂)ᵣ-O]ₛ-H with r = 1, 2, 3, 4, 5 or 6 and s = 1, 2, 3, 4, 5 or 6;
R¹⁰ means aryl';
R¹¹ means aryl' or OR²⁵;
R¹⁸ means H or methyl;
R¹⁹ means H, aryl¹ or in each case unsubstituted methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl;
R²¹, R²², R²³ and R²⁴ mutually independently mean H, fluorine, chlorine, bromine, iodine or OR²⁸;
R²⁵ means H, methyl, ethyl, n-propyl, 2-propyl, n-butyl, iso-butyl, sec.-butyl, tert.-butyl, n-amyl, iso-amyl, sec.-amyl, n-hexyl, iso-hexyl or sec.-hexyl, wherein R²⁵ does not mean H if, at the same time, R¹ means aryl and R² means alkyl;
R²⁸ means H, methyl or ethyl;
heterocyclyl means furan-2-yl,- furan-3-yl, thien-2-yl, thien-3-yl, pyridin-2-yl, pyridin-3-yl or pyridin-4-yl, wherein furanyl, thienyl and pyridinyl are in each case unsubstituted or monosubstituted or identically or differently polysubstituted with a substituent selected from the group consisting of F, Cl, Br, I, -CN, -NO₂, -CO₂H and -CO₂-alkyl;
aryl' means aryl¹ , aryl² or aryl³;
aryl¹ denotes 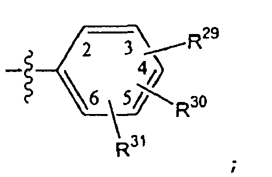
aryl² denotes 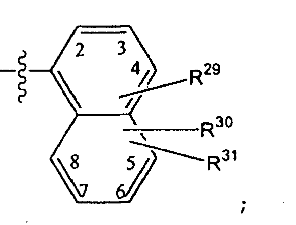
aryl³ denotes 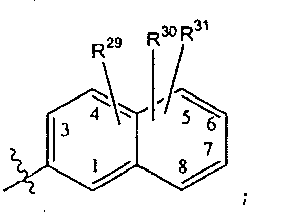
R²⁹, R³⁰ and R³¹ mutually independently mean H, C₁₋₆- [alk], (C₃₋₈-[cycloalk], - (C₁₋₆-alkyl)-C₃₋₈-[cycloalk], [Ar], - (C₁₋₆-alkyl)-[Ar], [het], -(C₁₋₆-alkyl)-[het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N-[Ar], - (C=O)R³⁷, 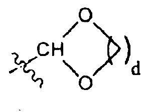 with d = 1, 2 or 3, or -(C=S)R³⁷ and may be in any desired ring position;
R³² and R³³ mutually independently mean H, -C₁₋₆-[alk], -C₃₋₈-[cycloalk], - (C₁₋₆-alkyl) -C₃₋₈-[cycloalk], -[Ar], - (C₁₋₆-alkyl)-[Ar], -[het], -(C₁₋₆-alkyl)-[het], (C=O)R³⁸, -[(CH₂)_{w}O]_{z}-H or -[(CH₂)_{w}-O]_{z}-C₁₋₆-[alk] with w = 1, 2, 3 or 4 and z = 1, 2, 3, 4 or 5;
R³⁴ means C₁₋₆-[alk], -CH₂-[Ar] or -(C=O)O-tert.-butyl;
R³⁵ and R³⁶ mutually independently mean C₁₋₆-[alk] or together denote -(CH₂)_{g}- with g = 4 or 5;
R³⁷ means H, -C₁₋₆-[alk], -C₃₋₈-[cycloalk], - (C₁₋₆-alkyl) -C₃₋₈-[cycloalk], -[Ar], - (C₁₋₆-alkyl)-[Ar], -[het], -(C₁₋₆-alkyl)-[het], -OR³⁹, -NH₂, -NHR³⁴, -NR³⁵R³⁶;
R³⁸ H, -C₁₋₆-[alk], -C₃₋₈-[cycloalk], - (C₁₋₆-alkyl)-C₃₋₈-[cycloalk], -[Ar], - (C₁₋₆-alkyl)-[Ar];
and
R³⁹ means H, C₁₋₆-[alk], -C₃₋₈- [cycloalk], - (C₁₋₆-alkyl)-C₃₋₈-[cycloalk], - [Ar], - (C₁₋₆-alkyl)-[Ar], -[het] or - (C₁₋₆-alkyl)-[het],
[alk] denotes an acyclic, saturated or unsaturated, branched or linear hydrocarbon residue which may be unsubstituted or monosubstituted or identically or differently polysubstituted,
wherein the substituents may be selected from the group consisting of F, Cl, Br, I, -CN, -NC, -NH₂, -NO₂, -SH, -OH, -CO₂H, -CO₂-alkyl,
[cycloalk] denotes a cyclic, saturated or unsaturated hydrocarbon residue which may be unsubstituted or monosubstituted or identically or differently polysubstituted and optionally benzo-fused,
wherein the substituents may be selected from the group consisting of F, Cl, Br, I, -CN, -NC, -NH₂, -NO₂, -SH, -OH, -CO₂H, -CO₂-alkyl,
[Ar] denotes an unsubstituted residue selected from the group consisting of phenyl, naphthyl, anthracenyl and biphenyl;
[het] denotes an unsubstituted heterocyclyl residue selected from the group consisting of pyrrolidinyl, tetrahydrofuryl, piperidinyl, piperazinyl and morpholinyl or denotes an unsubstituted heteroaryl residue selected from the group consisting of pyrrolyl, pyrazolyl, imidazolyl, pyridazinyl, pyrimidinyl, pyrazinyl, furanyl, thienyl and pyridinyl,
for the production of a medicament for the treatment of pain.

12. Use of a compound of the general structure (II), 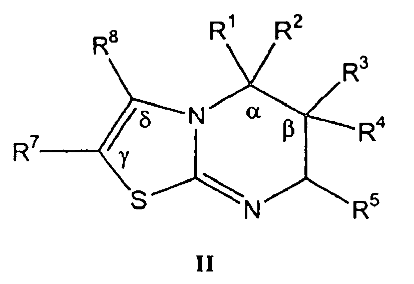 in the form as prepared or in the form of the acid(s) or base(s) thereof or in the form of one of the salts thereof, in particular the physiologically acceptable salts, or in the form of one of the solvates thereof, in particular the hydrates; in the form of the racemate thereof, the pure stereoisomers, in particular enantiomers or diastereomers, or in the form of mixtures of the stereoisomers, in particular the enantiomers or diastereomers, in any desired mixing ratio; in which R¹, R², R³, R⁴, R⁵, R⁷ and R⁸ are defined as in claim 11; for the production of a medicament for the treatment and/or prevention of epilepsy, schizophrenia, neurodegenerative diseases, in particular Alzheimer's disease, Huntington's chorea and Parkinson's disease, of cerebral ischaemic episodes and infarcts, of psychoses brought about by elevated amino acid levels, cerebral oedema, insufficiency states of the central nervous system, in particular in hypoxia and anoxia, of AIDS dementia, of encephalomyelitis, of Tourette's syndrome, of perinatal asphyxia and/or in tinnitus.

13. A pharmaceutical composition which contains at least one compound of the general structure (II), 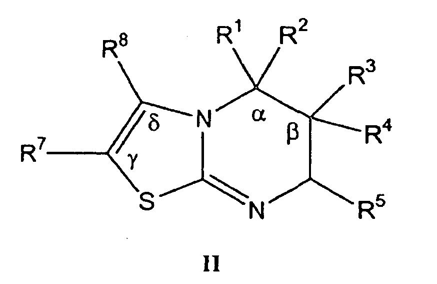 in the form as prepared or in the form of the acid(s) or base(s) thereof or in the form of one of the salts thereof, in particular the physiologically acceptable salts, or in the form of one of the solvates thereof, in particular the hydrates;
in the form of the racemate thereof, the pure stereoisomers, in particular enantiomers or diastereomers, or in the form of mixtures of the stereoisomers, in particular the enantiomers or diastereomers, in any desired mixing ratio; in which R¹, R², R³, R⁴, R⁵, R⁷ and R⁸ are defined as in claim 1; together with at least one pharmaceutical auxiliary substance.

## Revendications

1. Composé de formule générale (II) 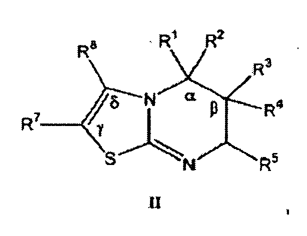 sous la forme représentée ou sous sa forme d'acide(s) ou de base(s) ou sous forme de l'un de ses sels, en particulier des sels physiologiquement acceptables, ou sous forme de l'un de ses produits de solvatation, en particulier des hydrates ;
sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;
formule dans laquelle
R¹ et R² représentent, indépendamment l'un de l'autre, H, un groupe O-R⁹, S-R¹⁰, [alk]-en C₁ à C₆, aryle' ou - (alkyle en C₁ à C₆) -aryle' , les substituants R²⁹, R³⁰ et R³¹ de aryle' représentant indépendamment les uns des autres H, un reste [alk] -en C₁ à C₆, F, Cl, Br, I, un groupe OH, un reste O-[alk] en C₁ à C₆), O-aryle¹ ou O-CH₂-aryle¹,
l'un des restes R¹ et R² représentant H et l'autre des restes R¹ et R² ne représentant pas H ou bien, au cas où l'un des restes R¹ et R² désigne un reste aryle', l'autre des restes R¹ et R² représente H ou un reste alkyle en C₁ à C₆,
R³ et R⁴ représentent H, un reste aryle' ou -CH₂-aryle' ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, l'un au moins des restes R³ et R⁴ représentant H,
ou bien
l'un des restes R¹ et R² forme conjointement avec l'un des restes R³ et R⁴ un groupe W, W étant un groupe α'-CH=CH-CH₂-β', α'-CH=CH-CH₂-CH₂-β', α'-O-(CH₂)ₘ-β' avec m = 2, 3, 4 ou 5, 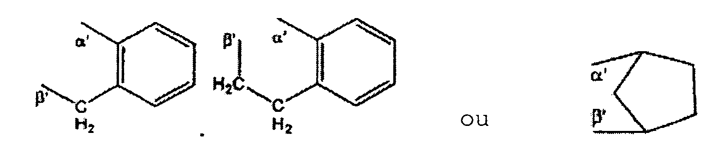 l'extrémité de W désignée par α' étant liée avec l'atome désigné par α du composé de formule générale (II) et l'extrémité de W désignée par β' étant liée à l'atome désigné par β du composé de formule générale (II), l'autre des restes R¹ et R² représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre des restes R³ et R⁴ représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle ;
R⁵ désigne un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle, ces restes étant chacun non substitués ou substitués une ou plusieurs fois identiques ou différentes avec un substituant choisi dans le groupe des substituants F, Cl, Br, I, -CN, -NC, -NO₂, -SH, -OH, -CO₂H et CO₂-alkyle ; aryle' ou -(CH₂)ₖ-aryle', k ayant la valeur 1, 2, 3 ou 4, hétérocyclyle ou C(=O)R¹¹ ;
R⁷ représente H, un reste aryle¹, OR¹⁸, S(O)_{q}R¹⁹, q ayant la valeur 0, 1 ou 2, ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle,
R⁸ représente H ou un reste aryle',
ou bien les restes R⁷ et R⁸ forment ensemble un groupe Y, Y représentant un groupe γ'-CR²¹=CR²²-CR²³=CR²⁴-δ' et l'extrémité de Y désignée par γ' est liée à l'atome désigné par γ de la formule générale (II) et l'extrémité de Y désignée par δ' est liée à l'atome désigné par δ de la formule générale (II) ;
R⁹ représente un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]s-H avec r = 1, 2, 3, 4, 5 ou 6 et s = 1, 2, 3, 4, 5 ou 6 ;
R¹⁰ représente un reste aryle' ;
R¹¹ représente un reste aryle' ou un groupe OR²⁵ ;
R¹⁸ représente H ou un reste méthyle ;
R¹⁹ représente H, un reste aryle¹ ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ;
R²¹, R²², R²³ et R²⁴ représentent, indépendamment les uns des autres, H, le fluor, le chlore, le brome, l'iode ou un groupe OR²⁸;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁸ représente H, un reste méthyle ou éthyle ;
hétérocyclyle désigne un groupe furanne-2-yle, furanne-3-yle, thién-2-yle, thién-3-yle, pyridine-2-yle, pyridine-3-yle ou pyridine-4-yle, les groupes furannyle, thiényle et pyridinyle étant chacun non substitués ou substitués une ou plusieurs fois identiques ou différentes avec un substituant choisi dans le groupe constitué de F, Cl, Br, I, -CN, -NO₂, -CO₂H ou -CO₂-alkyle ;
aryle' désigne un groupe aryle¹, aryle² ou aryle³ ;
aryle¹ représente 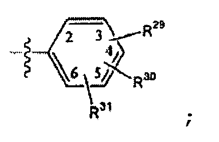
aryle² représente 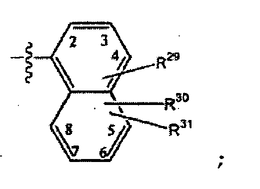
aryle³ représente 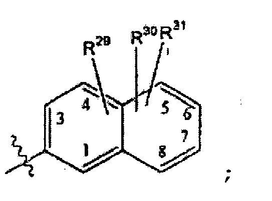
R²⁹, R³⁰ et R³¹ représentent, indépendamment les uns des autres, H, un reste [alk] -en C₁ à C₆, [cycloalk]-en C₃ à C₈, -[cycloalkyle en C₁ à C₆)-[cycloalk]-en C₃ à C₈, [Ar], -(alkyle en C₁ à C₆)-[Ar], [Het] , -(alkyle en C₁ à C₆)-[Het], F, Cl, Br, I, -CN, -NC, -OR³², -SR³³, -NO, -NO₂, -NH₂, -NHR³⁴, NR³⁵R³⁶, -N-OH, -N-O-[alk en C₁ à C₆], -NHNH₂, -N=N-[Ar], -(C=O)R³⁷, 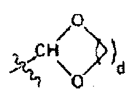 avec d = 1, 2 ou 3, ou -(C=S)R³⁷ et peuvent occuper n'importe quelle position sur le noyau ;
R³² et R³³ représentent, indépendamment l'un de l'autre, H, un reste -[alk en C₁ à C₆], -[cycloalk en C₃ à C₈], -(alkyle en C₁ à C₆) -[cycloalk en C₃ à C₈], -[Ar], -(alkyle en C₁ à C₆) -[Ar], -[Het], -(alkyle en C₁ à C₆)-[Het], (C=O)R³⁸, -[(CH₂)_{w}-O]_{z}-H ou -[(CH₂)_{w}-O]_{z}-[alk en C₁ à C₆] avec w = 1, 2, 3 ou 4 et z = 1, 2, 3, 4 ou 5 ;
R³⁴ représente un reste [alk]-en C₁ à C₆, -CH₂-[Ar] ou -(C=O)O-tertiobutyle ;
R³⁵ et R³⁶ représentent, indépendamment l'un de l'autre, un reste -[alk]-en C₁ à C₆, ou bien forment ensemble un reste -(CH₂)g- avec g = 4 ou 5 ;
R³⁷ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar], -[Het], -(alkyle en C₁ à C₆)-[Het], -OR³⁹, -NH₂, -NHR³⁴, -NR³⁵R³⁶ ;
R³⁸ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar];
et
R³⁹ représente H, un reste [alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar], -[Het] ou -(alkyle en C₁ à C₆)-[Het],
[alk] désignant un reste hydrocarboné acyclique, saturé ou non saturé, ramifié ou non ramifié, qui peut être non substitué ou substitué une ou plusieurs fois identiques ou différentes,
les substituants pouvant être choisis dans le groupe constitué de F, Cl, Br, I, -CN, -NC, -NH₂, -NO₂, -SH, -OH, -CO₂H, -CO₂-alkyle,
[cycloalk] désignant un reste hydrocarboné cyclique, saturé ou non saturé, qui peut être non substitué ou substitué une ou plusieurs fois identiques ou différentes et éventuellement condensé au benzène,
les substituants pouvant être choisis dans le groupe constitué de F, Cl, Br, I, -CN, -NC, -NH₂, -NO₂, -SH, -OH, -CO₂H, -CO₂-alkyle,
[Ar] désignant un reste non substitué choisi dans le groupe constitué de phényle, naphtyle, anthracényle et biphényle ;
[Het] désignant un reste hétérocyclyle non substitué choisi dans le groupe constitué des restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle,
les composés
3,4-dihydro-2-(4-nitrophényl)-4-phényl-2H-pyrimido[2,1-b]benzothiazole et
3,4-dihydro-4-(4-méthylphényl)-2-(4-nitrophényl)-2H-pyrimido[2,1-b]benzothiazole
étant exclus.

2. Composé suivant la revendication 1, **caractérisé en ce que**
R¹ et R² représentent, indépendamment l'un de l'autre, H, un groupe O-R⁹, S-R¹⁰, un reste non substitué ou substitué une ou plusieurs fois identiques ou différentes, méthyle, éthyle, n-propyle, 2-propyle, n-butyle, tertiobutyle ou n-hexyle, les substituants étant choisis dans le groupe constitué de F, Cl, Br, I, -CN, -NC, -NH₂, -NO₂, -SH et -OH ; un reste aryle' ou -CH₂-aryle', les substituants R²⁹, R³⁰ et R³¹ de aryle' représentant, indépendamment les uns des autres, H, un reste méthyle, éthyle, 2-propyle, n-butyle, tertiobutyle, n-hexyle, F, Cl, Br, I, OH, O-méthyle, O-éthyle,
l'un des restes R¹ et R² représentant H et l'autre des restes R¹ et R² ne représentant pas H ou bien, au cas où l'un des restes R¹ et R² désigne un reste aryle', l'autre des restes R¹ et R² représente H ou un reste méthyle, éthyle, n-propyle, 2-propyle, n-butyle, tertiobutyle ou n-hexyle,
R³ et R⁴ représentent H, un reste méthyle ou aryle¹, les substituants R²⁹, R³⁰ et R³¹ de aryle¹ représentant indépendamment les uns des autres H, un reste méthyle ou O-méthyle, l'un au moins des restes R³ et R⁴ représentant H,
ou bien
l'un des restes R¹ et R² forme conjointement avec l'un des restes R³ et R⁴ un groupe W, W étant un groupe α'-CH=CH-CH₂-β', α'-CH=CH-CH₂-CH₂-β',α'-O-(CH₂)ₘ-β' avec m = 2, 3, 4 ou 5, 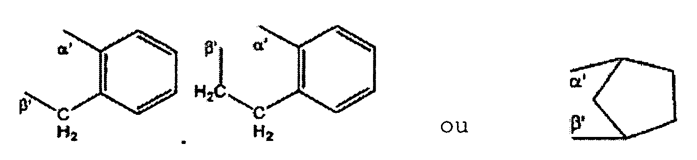 l'extrémité de W désignée par α' étant liée avec l'atome désigné par α du composé de formule générale (II) et l'extrémité de W désignée par β' étant liée à l'atome désigné par β du composé de formule générale (II), l'autre des restes R¹ et R² et l'autre des restes R³ et R⁴ représentant chacun H ;
R⁵ est un reste méthyle, éthyle, n-propyle, 2-propyle, n-butyle, isobutyle, sec.-butyle, tertiobutyle, -(CH₂)₄-OH, cyclopropyle, le reste cyclopropyle étant non substitué ou substitué une fois avec un groupe C(=O)OH, C(=O)O-méthyle ou C(=O)O-éthyle, un reste cyclopentyle, cyclohexyle, aryle¹ ou -(CH₂)ₖ-aryle¹, les substituants R²⁹, R³⁰ et R³¹ de aryle¹ représentant, indépendamment les uns des autres, H, un groupe -OH, -O-méthyle, O-C₆H₅, CH₃, CF₃ ou C(=O)OH et k a la valeur 1 ou 2, un reste hétérocyclyle ou C(=O)R¹¹;
R⁷ désigne H, un reste aryle¹ avec R²⁹, R³⁰ et R³¹ représentant H, un groupe OH, S(O)_{q}R¹⁹, q étant égal à 0 ou à 2, ou un reste méthyle, éthyle, n-propyle, 2-propyle, n-butyle, isobutyle, sec.-butyle ou tertiobutyle,
R⁸ représente H, un reste aryle¹, les substituants R²⁹, R³⁰ et R³¹ du reste aryle¹ représentant, indépendamment les uns des autres, H, un radical méthyle ou chloro, ou bien un reste aryle³ pour lequel R²⁹, R³⁰ et R³¹ représentent H,
ou bien
les restes R⁷ et R⁸ forment ensemble un groupe Y, Y représentant un groupe γ'-CR²¹=CR²²-CR²³=CR²⁴-δ' et l'extrémité de Y désignée par γ' est liée à l'atome désigné par γ de la formule générale (II) et l'extrémité de Y désignée par δ' est liée à l'atome désigné par δ de la formule générale (II) ;
R⁹ représente un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclopentyle ou cyclohexyle ou bien un groupe -[(CH₂)ᵣ-O]ₛ-H avec r = 1, 2 ou 3 et s = 1 ou 2 ;
R¹⁰ est un reste aryle¹ ;
R¹¹ est un reste aryle¹ pour lequel R²⁹, R³⁰ et R³¹ représentent H ou un groupe OR²⁵ ;
R¹⁹ est un reste méthyle ou aryle¹, l'un des substituants R²⁹, R³⁰ et R³¹ de aryle¹ représente H ou un groupe -NO₂ et les deux autres substituants de aryle¹ parmi les substituants R²⁹, R³⁰ et R³¹ représentent H,
R²¹ et R²³ représentent H ;
R²² représente H, le fluor ou un groupe OR²⁸ ;
R²⁴ représente H ou le chlore;
R²⁵ représente H, un reste méthyle ou éthyle, R²⁵ ne désignant par H lorsque R¹ est un reste aryle en même temps que R² est un reste alkyle ;
R²⁸ représente un reste aryle ou éthyle ; et
hétérocyclyle désigne un groupe furanne-2-yle, furanne-3-yle, thién-2-yle, thién-3-yle, pyridine-2-yle, pyridine-3-yle ou pyridine-4-yle, les groupes furannyle, thiényle et pyridinyle étant chacun non substitués ou substitués une ou plusieurs fois identiques ou différentes par un groupe -NO₂, -CH₃ ou C(=O)OH.

3. Composé suivant la revendication 1 ou 2,
**caractérisé en ce que**
R¹ et R² représentent indépendamment l'un de l'autre H, un groupe O-CH₂-CH₂-OH, O-cyclohexyle, S-phényle, un reste méthyle, phényle, 3-fluorophényle, 3-bromophényle, 4-bromophényle, 4-chlorophényle, 4-fluorophényle, 3-méthylphényle, 4-hydroxyphényle, 4-méthoxyphényle, 2,4-diméthylphényle, 3,4-diméthoxyphényle, 2,3,4-triméthoxyphényle, 2-naphtyle ou -CH₂-phényle,
R³ et R⁴ représentent H, un reste méthyle ou 4-méthoxyphényle, l'un au moins des restes R³ et R⁴ représentant H,
ou bien
l'un des restes R¹ et R² forme conjointement avec l'un des restes R³ et R⁴ un groupe W, W étant un groupe α'-CH=CH-CH₂-β', α'-CH=CH-CH₂-CH₂-β', α'-O-(CH₂)ₘ-β' avec m = 2, 3, 4 ou 5, 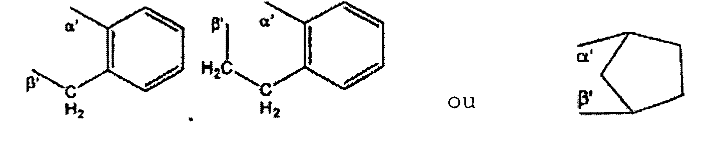 l'extrémité de W désignée par α' est liée à l'atome désigné par α du composé de formule générale (II) et l'extrémité de W désignée par β' est liée à l'atome désigné par β du composé de formule générale (II), l'autre des restes R¹ et R² et l'autre des restes R³ et R⁴ représentent chacun H ;
R⁵ est un reste n-propyle, n-butyle, tertiobutyle, -(CH₂)₄-OH, cyclopropyle, ester éthylique d'acide cycloprop-2-yl-1-carboxylique, cyclohexyle, 4-trifluorophényle, 4-phénoxyphényle, 2-hydroxy-3-méthoxyphényle, 4-hydroxy-3-méthoxyphényle, 3-carboxy-2-hydroxyphényle, -(CH₂)₂-phényle, 5-carboxyfuranne-2-yle, 5-méthylfuranne-2-yle, 5-nitrofuranne-2-yle, 5-nitrothién-2-yle, pyridine-2-yle, pyridine-3-yle, C (=O) -phényle, C (=O) OH ou C (=O) O-éthyle, R⁵ ne représentant pas C(=O)OH lorsque R¹ est un reste aryle en même temps que R² est un reste alkyle ;
R⁷ représente H, un reste phényle, OH, -S-méthyle, -SO₂-(4-nitrophényle) ou tertiobutyle,
R⁸ est un reste 4-chlorophényle, 4-méthylphényle ou 2-naphtyle
ou bien
les restes R⁷ et R⁸ forment ensemble un groupe Y, Y désignant un groupe γ'-CR²¹=CH-CH=CH-δ' et l'extrémité de Y désignée par γ' est liée à l'atome désigné par γ de la formule générale (II) et l'extrémité de Y désignée par δ' est liée à l'atome désigné par δ de la formule générale (II) ; et
R²¹ représente le fluor, un reste méthoxy ou éthoxy.

4. Composé selon l'une quelconque des revendications 1 à 3, **caractérisé en ce qu'**il est choisi parmi les composés suivants :
• 2-(4-nitrophénylsulfonyl)-5-phénylsulfanyl-7-pyridine-2-yl-6,7-dihydro-5H-thiazolo[3,2-a]-pyrimidine
• 3-(4-chlorophényl)-5-phénylsulfanyl-7-pyridine-2-yl-6,7-dihydro-5H-thiazolo[3,2-a]-pyrimidine
• 5-phénylsulfanyl-7-pyridine-2-yl-3-p-tolyl-6,7-dihydro-5H-thiazolo[3,2-a]pyrimidine
• 7-méthoxy-4-phénylsulfanyl-2-pyridine-2-yl-3,4-dihydro-2H-9-thia-1,4a-diazafluorène
• 7-éthoxy-4-phénylsulfanyl-2-pyridine-2-yl-3,4-dihydro-2H-9-thia-1,4a-diazafluorène
• 7-fluoro-4-phénylsulfanyl-2-pyridine-2-yl-3,4-dihydro-2H-9-thia-1,4a-diazafluorène
• 3-naphtalène-2-yl-5-phénylsulfanyl-7-pyridine-2-yl-6,7-dihydro-5H-thiazolo[3,2-a]pyrimidine.

5. Procédé de production de composés de formule générale (II) ou de leurs sels pharmaceutiquement acceptables 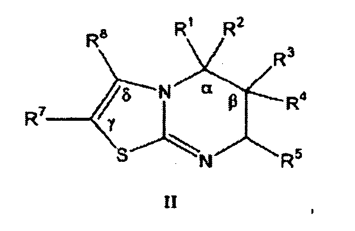 formule dans laquelle R¹, R², R³, R⁴, R⁵, R⁷ et R⁸ sont tels que définis dans la revendication 1,
**caractérisé en ce qu'**on fait réagir une thiazolamine de formule générale (VI) 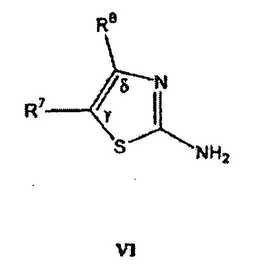 dans laquelle
R⁷ et R⁸ sont tels que définis ci-dessus, sous réserve que lorsque R⁷ et R⁸ forment un groupe Y, l'extrémité de Y désignée par γ' soit liée à l'atome désigné par γ de la thiazolamine de formule générale (VI) et que l'extrémité de Y désignée par δ' soit liée à l'atome désigné par δ de la thiazolamine de formule générale (VI),
avec un aldéhyde de formule générale (IV) 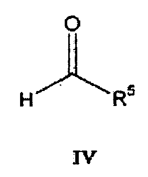 dans laquelle
R⁵ est tel que défini ci-dessus, et une oléfine de formule générale (V) 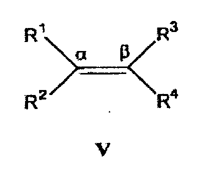 dans laquelle
R¹, R², R³ et R⁴ sont tels que définis ci-dessus, sous réserve que, lorsqu'un des restes R¹ et R² forme un groupe W conjointement avec l'un des restes R³ et R⁴, l'extrémité de W désignée par α' soit liée à l'atome de carbone désigné par α de l'oléfine de formule générale (V) et que l'extrémité de W désignée par β' soit liée à l'atome de carbone β de l'oléfine de formule générale (V),
en présence d'un acide.

6. Procédé suivant la revendication 5, **caractérisé en ce que** la réaction de l'hétérocyclylamine de formule générale (VI) avec l'aldéhyde de formule générale (IV) et l'oléfine de formule générale (V) est conduite selon une technique opératoire en récipient unique.

7. Procédé selon la revendication 5 ou 6, **caractérisé en ce que** l'acide est l'acide trifluoracétique.

8. Procédé selon l'une quelconque des revendications 5 à 7, **caractérisé en ce que** la réaction est conduite dans un solvant organique à une température de 0 à 100°C et pendant un temps de réaction de 0,25 à 12 heures.

9. Procédé selon l'une des revendications 5 à 8, **caractérisé en ce que** la réaction est conduite à une température de 15 à 40°C.

10. Médicament contenant au moins un composé de formule générale (II) 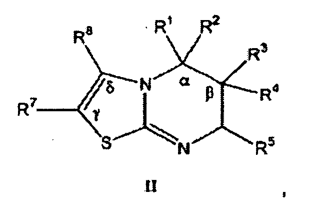 sous la forme représentée ou sous sa forme d'acide(s) ou de base(s) ou sous forme de l'un de ses sels, en particulier des sels physiologiquement acceptables, ou sous forme de l'un de ses produits de solvatation, en particulier des hydrates ; sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque; formule dans laquelle
R¹ et R² représentent, indépendamment l'un de l'autre, H, un groupe O-R⁹, S-R¹⁰, [alk]-en C₁ à C₆, aryle' ou -(alkyle en C₁ à C₆)-aryle', les substituants R²⁹, R³⁰ et R³¹ de aryle' représentant indépendamment les uns des autres H, un reste [alk]-en C₁ à C₆, F, Cl, Br, I, un groupe OH, un reste O-[alk] en C₁ à C₆, O-aryle¹ ou O-CH₂-aryle¹,
l'un des restes R¹ et R² représentant H et l'autre des restes R¹ et R² ne représentant pas H ou bien, au cas où l'un des restes R¹ et R² désigne un reste aryle', l'autre des restes R¹ et R² représente H ou un reste alkyle en C₁ à C₆,
R³ et R⁴ représentent H, un reste aryle' ou -CH₂-aryle' ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle,
l'un au moins des restes R³ et R⁴ représentant H,
ou bien
l'un des restes R¹ et R² forme conjointement avec l'un des restes R³ et R⁴ un groupe W, W étant un groupe α'-CH=CH-CH₂-β', α'-CH=CH-CH₂-CH₂-β', α'-O-(CH₂)ₘ-β' avec m = 2, 3, 4 ou 5, 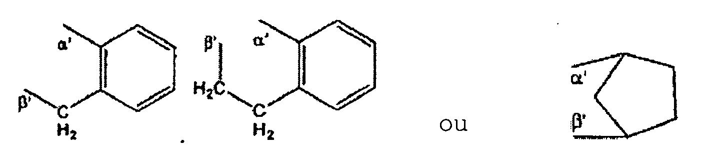 l'extrémité de W désignée par α' étant liée avec l'atome désigné par α du composé de formule générale (II) et l'extrémité de W désignée par β' étant liée à l'atome désigné par β du composé de formule, générale (II), l'autre des restes R¹ et R² représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre des restes R³ et R⁴ représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle ;
R⁵ désigne un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle, ces restes étant chacun non substitués ou substitués une ou plusieurs fois identiques ou différentes avec un substituant choisi dans le groupe des substituants F, Cl, Br, I, -CN, -NC, -NO₂, -SH, -OH, -CO₂H et CO₂-alkyle ; aryle' ou -(CH₂)ₖ-aryle', k ayant la valeur 1, 2, 3 ou 4, hétérocyclyle ou C(=O)R¹¹ ;
R⁷ représente H, un reste aryle¹, OR¹⁸, S(O)_{q}R¹⁹, q ayant la valeur 0, 1 ou 2, ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle,
R⁸ représente H ou un reste aryle',
ou bien les restes R⁷ et R⁸ forment ensemble un groupe Y, Y représentant un groupe γ'-CR²¹=CR²²-CR²³=CR²⁴-δ' et l'extrémité de Y désignée par γ' est liée à l'atome désigné par γ de la formule générale (II) et l'extrémité de Y désignée par δ' est liée à l'atome désigné par δ de la formule générale (II) ;
R⁹ représente un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]ₛ-H avec r = 1, 2, 3, 4, 5 ou 6 et s = 1, 2, 3, 4, 5 ou 6 ;
R¹⁰ représente un reste aryle';
R¹¹ représente un reste aryle' ou un groupe OR²⁵ ;
R¹⁸ représente H ou un reste méthyle ;
R¹⁹ représente H, un reste aryle¹ ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ;
R²¹, R²², R²³ et R²⁴ représentent, indépendamment les uns des autres, H, le fluor, le chlore, le brome, l'iode ou un groupe OR²⁸ ;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁸ représente H, un reste méthyle ou éthyle ;
hétérocyclyle désigne un groupe furanne-2-yle, furanne-3-yle, thién-2-yle, thién-3-yle, pyridine-2-yle, pyridine-3-yle ou pyridine-4-yle, les groupes furannyle, thiényle et pyridinyle étant chacun non substitués ou substitués une ou plusieurs fois identiques ou différentes avec un substituant choisi dans le groupe constitué de F, Cl, Br, I, -CN, -NO₂, -CO₂H ou -CO₂-alkyle ;
aryle' désigne un groupe aryle¹, aryle² ou aryle³;
aryle¹ représente 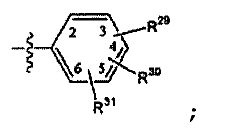
aryle² représente 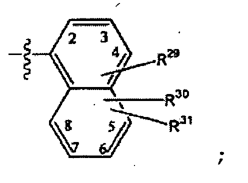
aryle³ représente 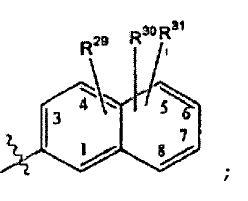
R²⁹, R³⁰ et R³¹ représentent, indépendamment les uns des autres, H, un reste [alk]-en C₁ à C₆, [cycloalk]-en C₃ à C₈, -(cycloalkyle en C₁ à C₆)-[cycloalk]-en C₃ à C₈, [Ar], -(alkyle en C₁ à C₆)-[Ar], [Het], -(alkyle en C₁ à C₆)-[Het], F, Cl, Br, I, -CN, -NC, -OR³², -SR³³, -NO, -NO₂, -NH₂, -NHR³⁴, NR³⁵R³⁶, -N-OH, -N-O-[alk en C₁ à C₆], -NHNH₂, -N=N-[Ar], -(C=O)R³⁷, 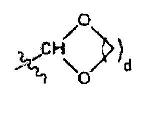 avec d = 1, 2 ou 3, ou -(C=S)R³⁷ et peuvent occuper n'importe quelle position sur le noyau ;
R³² et R³³ représentent, indépendamment l'un de l'autre, H, un reste -[alk en C₁ à C₆], -[cycloalk en C₃ à C₈], -(alkyle en C₁ à C₆)-[cycloalk en C₃ à C₈], - [Ar], -(alkyle en C₁ à C₆)-[Ar], -[Het], -(alkyle en C₁ à C₆)-[Het], (C=O)R³⁸, -[(CH₂)_{w}-O]_{z}-H ou -[(CH₂)_{w}-O]_{z}-[alk en C₁ à C_{6]} avec w = 1, 2, 3 ou 4 et z = 1, 2, 3, 4 ou 5 ;
R³⁴ représente un reste [alk]-en C₁ à C₆, -CH₂-[Ar] ou -(C=O)O-tertiobutyle ;
R³⁵ et R³⁶ représentent, indépendamment l'un de l'autre, un reste -[alk]-en C₁ à C₆, ou bien forment ensemble un reste -(CH₂)_{g}- avec g = 4 ou 5 ;
R³⁷ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar], -[Het], -(alkyle en C₁ à C₆)-[Het], -OR³⁹, -NH₂, -NHR³⁴, -NR³⁵R³⁶ ;
R³⁸ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar] ;
et
R³⁹ représente H, un reste [alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar], -[Het] ou - (alkyle en C₁ à C₆)-[Het],
[alk] désignant un reste hydrocarboné acyclique, saturé ou non saturé, ramifié ou non ramifié, qui peut être non substitué ou substitué une ou plusieurs fois identiques ou différentes,
les substituants pouvant être choisis dans le groupe constitué de F, Cl, Br, I, -CN, -NC, -NH₂, -NO₂, -SH, -OH, -CO₂H, -CO₂-alkyle,
[cycloalk] désignant un reste hydrocarboné cyclique, saturé ou non saturé, qui peut être non substitué ou substitué une ou plusieurs fois identiques ou différentes et éventuellement condensé au benzène,
les substituants pouvant être choisis dans le groupe constitué de F, Cl, Br, I, -CN, -NC, -NH₂, -NO₂, -SH, -OH, -CO₂H, -CO₂-alkyle,
[Ar] désignant un reste non substitué choisi dans le groupe constitué de phényle, naphtyle, anthracényle et biphényle ;
[Het] désignant un reste hétérocyclyle non substitué choisi dans le groupe constitué des restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle.

11. Utilisation d'un composé de formule générale (II) 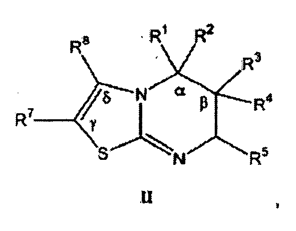 sous la forme représentée ou sous sa forme d'acide(s) ou de base(s) ou sous forme de l'un de ses sels, en particulier des sels physiologiquement acceptables, ou sous forme de l'un de ses produits de solvatation, en particulier des hydrates ;
sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; formule dans laquelle
R¹ et R² représentent, indépendamment l'un de l'autre, H, un groupe O-R⁹, S-R¹⁰, [alk]-en C₁ à C₆, aryle' ou -(alkyle en C₁ à C₆) -aryle' , les substituants R²⁹, R³⁰ et R³¹ de aryle' représentant indépendamment les uns des autres H, un reste [alk]-en C₁ à C₆, F, Cl, Br, I, un groupe OH, un reste O-[alk] en C₁ à C₆, O-aryle¹ ou O-CH₂-aryle¹,
l'un des restes R¹ et R² représentant H et l'autre des restes R¹ et R² ne représentant pas H ou bien, au cas où l'un des restes R¹ et R² désigne un reste aryle', l'autre des restes R¹ et R² représente H ou un reste alkyle en C₁ à C₆,
R³ et R⁴ représentent H, un reste aryle' ou -CH₂-aryle' ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle,
l'un au moins des restes R³ et R⁴ représentant H,
ou bien
l'un des restes R¹ et R² forme conjointement avec l'un des restes R³ et R⁴ un groupe W, W étant un groupe α'-CH=CH-CH₂-β', α'-CH=CH-CH₂-CH₂-β', α'-O-(CH₂)ₘ-β' avec m = 2, 3, 4 ou 5, 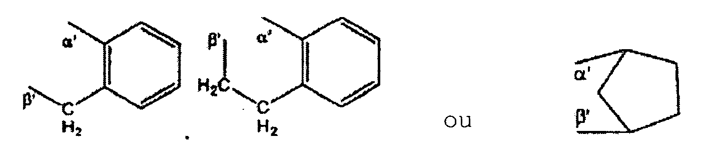 l'extrémité de W désignée par α' étant liée avec l'atome désigné par α du composé de formule générale (II) et l'extrémité de W désignée par β' étant liée à l'atome désigné par β du composé de formule générale (II), l'autre des restes R¹ et R² représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre des restes R³ et R⁴ représente H ou un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle ;
R⁵ désigne un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle, ces restes étant chacun non substitués ou substitués une ou plusieurs fois identiques ou différentes avec un substituant choisi dans le groupe des substituants F, Cl, Br, I, -CN, -NC, -NO₂, -SH, -OH, -CO₂H et CO₂-alkyle ; aryle' ou -(CH₂)ₖ-aryle', k ayant la valeur 1, 2, 3 ou 4, hétérocyclyle ou C(=O)R¹¹ ;
R⁷ représente H, un reste aryle¹, OR¹⁸, S(O)_{q}R¹⁹, q ayant la valeur 0, 1 ou 2, ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle,
R⁸ représente H ou un reste aryle',
ou bien les restes R⁷ et R⁸ forment ensemble un groupe Y, Y représentant un groupe γ'-CR²¹=CR²²-CR²³=CR²⁴-δ' et l'extrémité de Y désignée par γ' est liée à l'atome désigné par γ de la formule générale (II) et l'extrémité de Y désignée par δ' est liée à l'atome désigné par δ de la formule générale (II) ;
R⁹ représente un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]ₛ-H avec r = 1, 2, 3, 4, 5 ou 6 et s = 1, 2, 3, 4, 5 ou 6 ;
R¹⁰ représente un reste aryle' ;
R¹¹ représente un reste aryle' ou un groupe OR²⁵ ;
R¹⁸ représente H ou un reste méthyle ;
R¹⁹ représente H, un reste aryle¹ ou un reste non substitué méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ;
R²¹, R²², R²³ et R²⁴ représentent, indépendamment les uns des autres, H, le fluor, le chlore, le brome, l'iode ou un groupe OR²⁸ ;;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁸ représente H, un reste méthyle ou éthyle ;
hétérocyclyle désigne un groupe furanne-2-yle, furanne-3-yle, thién-2-yle, thién-3-yle, pyridine-2-yle, pyridine-3-yle ou pyridine-4-yle, les groupes furannyle, thiényle et pyridinyle étant chacun non substitués ou substitués une ou plusieurs fois identiques ou différentes avec un substituant choisi dans le groupe constitué de F, Cl, Br, I, -CN, -NO₂, -CO₂H ou -CO₂-alkyle ;
aryle' désigne un groupe aryle¹, aryle² ou aryle³ ;
aryle¹ représente 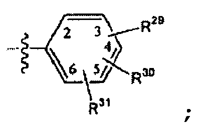
aryle² représente 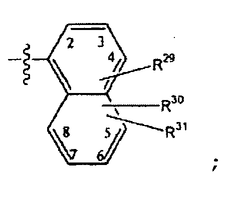
aryle³ représente 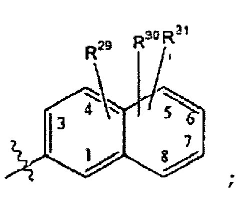
R ²⁹ R³⁰ et R³¹ représentent, indépendamment les uns des autres, H, un reste [alk]-en C₁ à C₆, [cycloalk]-en C₃ à C₈, -(cycloalkyle en C₁ à C₆)-[cycloalk] -en C₃ à C₈, [Ar], -(alkyle en C₁ à C₆)-[Ar], [Het], -(alkyle en C₁ à C₆)-[Het], F, Cl, Br, I, -CN, -NC, -OR³², -SR³³, -NO, -NO₂, -NH₂, -NHR³⁴, NR³⁵R³⁶, -N-OH, -N-O-[alk en C₁ à C₆], -NHNH₂, -N=N-[Ar], -(C=O)R³⁷, 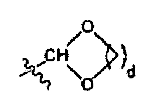 avec d = 1, 2 ou 3, ou -(C=S)R³⁷ et peuvent occuper n'importe quelle position sur le noyau ;
R³² et R³³ représentent, indépendamment l'un de l'autre, H, un reste -[alk en C₁ à C₆], -[cycloalk en C₃ à C₈], -(alkyle en C₁ à C₆)-[cycloalk en C₃ à C₈], -[Ar], -(alkyle en C₁ à C₆)-[Ar], -[Het], -(alkyle en C₁ à C₆)-[Het], (C=O)R³⁸, -[(CH₂)_{w}-O]_{z}-H ou -[(CH₂)_{w}-O]_{z}-[alk en C₁ à C₆] avec w = 1, 2, 3 ou 4 et z = 1, 2, 3, 4 ou 5 ;
R³⁴ représente un reste [alk]-en C₁ à C₆, -CH_{z}-[Ar] ou -(C=O)O-tertiobutyle ;
R³⁵ et R³⁶ représentent, indépendamment l'un de l'autre, un reste -[alk]-en C₁ à C₆, ou bien forment ensemble un reste -(CH₂)_{g}- avec g = 4 ou 5 ;
R³⁷ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar], -[Het], -(alkyle en C₁ à C₆)-[Het], -OR³⁹, -NH₂, -NHR³⁴, -NR³⁵R³⁶;
R³⁸ représente H, un reste -[alk]-en C₁ à C₆, -[cycloalk]-en C₃ à C₈, -(alkyle en C₁ à C₆)-[cycloalk en C₃ à C₈], -[Ar], -(alkyle en C₁ à C₆)-[Ar] ;
et
R³⁹ représente H, un reste [alk]-en C₁ à C₆, -[cycloalk]-en C₃ à C₈, -(alkyle en C₁ à C₆)-[cycloalk en C₃ à C₈],
-[Ar], -(alkyle en C₁ à C₆)-[Ar], -[Het] ou - (alkyle en C₁ à C₆)-[Het],
[alk] désignant un reste hydrocarboné acyclique, saturé ou non saturé, ramifié ou non ramifié, qui peut être non substitué ou substitué une ou plusieurs fois identiques ou différentes,
les substituants pouvant être choisis dans le groupe constitué de F, Cl, Br, I, -CN, -NC, -NH₂, -NO₂, -SH, -OH, -CO₂H, -CO₂-alkyle,
[cycloalk] désignant un reste hydrocarboné cyclique, saturé ou non saturé, qui peut être non substitué ou substitué une
ou plusieurs fois identiques ou différentes et éventuellement condensé au benzène,
les substituants pouvant être choisis dans le groupe constitué de F, Cl, Br, I, -CN, -NC, -NH₂, -NO₂, -SH, -OH, -CO₂H, -CO₂-alkyle,
[Ar] désignant un reste non substitué choisi dans le groupe constitué de phényle, naphtyle, anthracényle et biphényle ;
[Het] désignant un reste hétérocyclyle non substitué choisi dans le groupe constitué des restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle,
pour la préparation d'un médicament destiné au traitement de la douleur.

12. Utilisation d'un composé de formule générale (II), 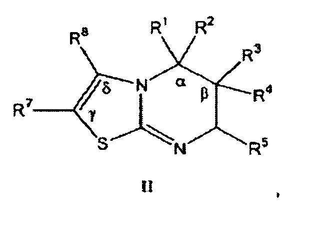 sous la forme représentée ou sous sa forme d'acide(s) ou de base (s) ou sous forme de l'un de ses sels, en particulier des sels physiologiquement acceptables, ou sous forme de l'un de ses produits de solvatation, en particulier des hydrates ; sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;
dans laquelle R¹, R², R³, R⁴, R⁵, R⁷ et R⁸ sont tels que définis dans la revendication 11 ;
pour la préparation d'un médicament destiné au traitement et/ou à la prophylaxie de l'épilepsie, la schizophrénie, des maladies neurodégénératives, en particulier la maladie d'Alzheimer, la maladie de Huntington et la maladie de Parkinson, d'ischémies cérébrales et d'infarctus cérébraux, de psychoses dues à un taux élevé d'acides aminés, d'oedème cérébral, d'états de sous-alimentation du système nerveux central, en particulier en cas d'hypoxies et d'anoxies, de démence liée au SIDA, d'encéphalomyélite, du syndrome de Tourette, de l'asphyxie périnatale et/ou en cas d'acouphènes.

13. Composition pharmaceutique, qui contient au moins un composé de formule générale (II) 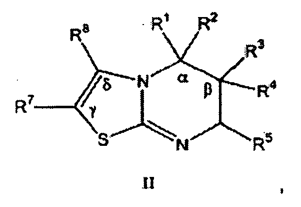 sous la forme représentée ou sous sa forme d'acide(s) ou de base(s) ou sous forme de l'un de ses sels, en particulier des sels physiologiquement acceptables, ou sous forme de l'un de ses produits de solvatation, en particulier des hydrates ; sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;
formule dans laquelle R¹, R², R³, R⁴, R⁵, R⁷ et R⁸ sont tels que définis dans la revendication 1 ; ainsi qu'au moins une substance auxiliaire pharmaceutique.
